# EUROPEAN PATENT APPLICATION

(11) **EP 3 106 168 A2**
(43) Date of publication of application: **21.12.2016**
(21) Application number: 16170595.9
(22) Date of filing: 30.11.2012
(51) Int. Cl.: A61K 31/7088, C12Q 1/68, A61P 35/00, A61K 31/7105, A61K 31/713, C12N 15/113, G01N 33/50

(54) **TARGETING MICRORNAS MIR-409-5P, MIR-379 AND MIR-154* TO TREAT PROSTATE CANCER BONE METASTASIS AND DRUG RESISTANT LUNG CANCER**

(30) Priority: 30.11.2011 US 201161565226 P
(62) Divisional of application: 12854115.8
(71) Applicant: Cedars-Sinai Medical Center, Los Angeles, CA 90048 (US)
(72) Inventor: CHUNG, Leland, W.K., Beverly Hills, CA 90210 (US); JOSSON, Sajni, Los Angeles, CA 90036 (US); GURURAJAN, Murali, Los Angeles, CA 90036 (US); JAIN, Anjali, Los Angeles, CA 90035 (US)
(74) Representative: Banford, Paul Clifford

(57) **Abstract**

The present invention describes methods of treating cancer, cancer metastasis, and drug resistant cancers using miRNA inhibitors; for example, inhibitors of miR-409-5p. Also described are methods of using the miRNA as biomarkers; for example, to predict responsiveness to a cancer drug, to detect a disease state of cancer.

## Description

### STATEMENT REGARDING FEDERALLY-SPONSORED RESEARCH

The U.S. Government has a paid-up license in this invention and the right in limited circumstances to require the patent owner to license others on reasonable terms as provided for by the terms of Grant No. CA122602 awarded by the National Institutes of Health.

### FIELD OF INVENTION

This invention relates to micro RNAs and cancer; particularly, to prostate cancer bone metastasis and drug resistant lung cancer.

### BACKGROUND

All publications herein are incorporated by reference to the same extent as if each individual publication or patent application was specifically and individually indicated to be incorporated by reference. The following description includes information that may be useful in understanding the present invention. It is not an admission that any of the information provided herein is prior art or relevant to the presently claimed invention, or that any publication specifically or implicitly referenced is prior art.

Small molecule tyrosine kinase inhibitors (TKIs) that compete for the ATP binding domain and inhibit the epidermal growth factor receptor (EGFR) pathway are approved for treating advanced stage non-small cell lung cancer (NSCLC) patients that have failed chemotherapy. The NSCLCs that are most responsive to EGFR-TKIs overexpress gene-amplified EGFR and/or a cluster of somatic mutations within the EGFR kinase domain. Despite a significant clinical benefit, majority of patients acquire resistance to EGFR-TKIs within an year and demonstrate disease progression. Identification of non-invasive biomarkers to identify such patients as they start progressing while on EGFR-TKIs would be extremely useful.

Bone is the second most common site of cancer metastasis, harboring over 70% of cancer metastases from prostate and breast cancers (1). Advanced-stage cancer patients develop bone metastases either with or without hormonal therapy, radiation therapy, chemotherapy, and immunotherapy, and currently there is no effective treatment. The pathogenesis of bone metastases remains poorly understood. Impairment of stroma cell function in the cancer microenvironment is believed to be an important step in tumor progression. Fibroblasts adjacent to cancer cells in the prostate are structurally and functionally different from normal fibroblast in the prostate (2,3). These cancer associated fibroblasts (CAFs), have different gene expression profiles from the normal fibroblasts. Cancer cells and stromal cells interact through physical contact, soluble factors and insoluble extra-cellular matrix factors. The CAFs have been shown to play a critical role in tumorigenesis. Studies show that loss of Transforming growth factor-beta type II receptor gene, in mouse fibroblast resulted in intraepithelial neoplasia in prostate (4). One of the mechanism by which cancer cells metastasize is by undergoing epithelial to mesenchymal transition (EMT). EMT is an conserved embryonic process where polarized immotile epithelial cells transition to apolar motile mesenchymal cells (5). EMT is associated with cancer migration, invasion and metastasis (6). The common feature of EMT is loss of E-cadherin and increase in vimentin and N-cadherin. In cancer, EMT allows benign tumors to infiltrate the surrounding tissue and metastasize to other organs.

MiRNAs are non-coding RNAs of 18-24 nucleotides that bind to sites of complementarity in the 3' untranslated regions of messenger RNAs and inhibit their translation (7). A single miRNA can target several mRNA and regulate cellular pathways and cell fate. Several miRNA have been dysregulated in cancer, some of these are oncogenic (oncomiR) or they function as tumor suppressors (8). MiRNA have also shown to play a role in metastasis and have been termed 'metastamirs' (9,10). Several miRNAs have been shown to be promote metastasis such as miR-10b in brain cancer (11), miR-21 in colorectal cancer (12), miR-184 in PCa (10,13). A few miRNA have been described which suppress PCa bone metastasis, such as miR-143, miR-145 and miR-203 (14). The inventors reported earlier that radiation treatment modulated microRNA expression in prostate cancer cells and that manipulation of microRNA expression sensitized cancer cells to radiation therapy. Several microRNAs have also been shown recently to promote or suppress metastasis. Notably, miR-200s have recently been shown to inhibit EMT and promote MET by direct targeting of E-cadherin transcriptional repressors *Zeb1* and *Zeb2* in breast cancers.

The lack of effective treatment for cancers, and particularly drug resistant cancers, along with the prevalence of bone metastasis shows a need in the art for additional therapies as well as biomarkers to discover and develop cancer therapeutics.

### SUMMARY OF INVENTION

The following embodiments and aspects thereof are described and illustrated in conjunction with compositions and methods which are meant to be exemplary and illustrative, not limiting in scope.

Various embodiments of the present invention provides for a method, comprising: providing a miRNA inhibitor; and administering the miRNA inhibitor to a subject in need of treatment for cancer, in need of treatment for cancer metastasis, or in need of lowering or treatment for cancer drug resistance to treat cancer, to treat cancer metastasis, or to lower or treat cancer drug resistance.

In various embodiments, the method can further comprise administering to the subject radiation treatment or chemotherapy treatment.

In various embodiments, the cancer can be prostate cancer, lung cancer, breast cancer, metastatic cancer, cancer metastasis to the bone, metastatic prostate cancer, metastatic lung cancer, or metastatic breast cancer.

In various embodiments, the miRNA inhibitor can be capable of inhibiting miR-379, miR-379*, miR-193b, miR-193b*, miR-409-5p, miR-409-3p, miR-154, and/or miR-154*. In particular embodiments, the miRNA inhibitor can be capable of inhibiting mature miR-379, miR-379*, miR-193b, miR-193b*, miR-409-5p, miR-409-3p, miR-154, and/or miR-154*.

In various embodiments, the miRNA inhibitor can be a siRNA directed against a mature miRNA.

In particular embodiments, the miRNA inhibitor can be a shRNA directed against a mature miRNA. In certain embodiments, the miRNA inhibitor can be encoded by a polynucleotide as disclosed by SEQ ID NO:1, SEQ ID NO:4, SEQ ID NO:7, or SEQ ID NO:10, and can comprise administering the polynucleotide.

In various embodiments, the miRNA inhibitor can be a shRNA or a siRNA capable of interfering the expression of SEQ ID NO:2, SEQ ID NO:3, SEQ ID NO:5, SEQ ID NO:6, SEQ ID NO:8, SEQ ID NO:9, SEQ ID NO: 11 or SEQ ID NO: 12.

Various embodiments of the present invention provide for a method, comprising: obtaining a biological sample from a subject; testing the biological sample for a relative increase, decrease, or steady expression of a miRNA; and associating a relative increase of the miRNA expression level with a lower likelihood of cancer drug responsiveness or associating a relative decrease or a steady expression level of the miRNA with a higher likelihood of drug responsiveness, associating a relative increase of the miRNA expression level with a higher likelihood of having a cancer drug resistant disease state or associating a relative decrease or a steady expression level of the miRNA with cancer drug susceptible disease state, or associating a relative increased expression the DLK1-miRNA with metastatic disease state or associating a relative decreased or a steady expression level of DLK1-miRNA with a non-metastatic disease state.

In various embodiments, the method can further comprise selecting a cancer drug to administer to the subject when a higher likelihood of drug responsiveness or a cancer drug susceptible disease state is detected. In various embodiments, the drug can be a tyrosine kinase inhibitor (TKI). In various embodiments, the TKI can be Erlotinib, Gefitinib, Apatinib, Cabozantinib, Canertinib, Crenolanib, Damnacanthal, Foretinib, Fostamatinib, Intedanib, Linifanib, Motesanib, Mubritinib, Vatalanib, or Vemurafenib.

In various embodiments, the miRNA can be miR-379, miR-379*, miR-193b, miR-193b*, miR-409-5p, miR-409-3p, miR-154, miR-154*, mature miR-379, mature miR-379*, mature miR-193b, mature miR-193b*, mature miR-409-5p, mature miR-409-3p, mature miR-154, mature miR-154*, SEQ ID NO:2, SEQ ID NO:3, SEQ ID NO:5, SEQ ID NO:6, SEQ ID NO:8, SEQ ID NO:9, SEQ ID NO: 11 or SEQ ID NO: 12.

In various embodiments, the cancer can be prostate cancer, lung cancer, breast cancer, metastatic cancer, cancer metastasis to the bone, metastatic prostate cancer, metastatic lung cancer or metastatic breast cancer.

Various embodiments of the present invention provide for a method, comprising: obtaining a biological sample from a subject; testing the biological sample for a relative increase, decrease, or steady expression of DLK1-DIO3 cluster/region; and associating a relative increased expression level of the DLK1-DIO3 cluster/region with a lower likelihood of drug responsiveness or associating a relative decreased or a steady expression level of the DLK1-DIO3 cluster/region with a higher likelihood of drug responsiveness, associating a relative increased expression level of the DLK1-DIO3 region with a cancer drug resistant disease state or associating a relative decreased or a steady expression level of the DLK1-DIO3 cluster/region with cancer drug susceptible disease state, or associating a relative increased expression the DLK1-DIO3 cluster/region with metastatic disease state or associating a relative decreased or a steady expression level of DLK1-DIO3 cluster/region with a non-metastatic disease state.

In various embodiments, the method can further comprise selecting a cancer drug to administer to the subject when a higher likelihood of drug responsiveness or a cancer drug susceptible disease state is detected. In various embodiments, the drug can be a tyrosine kinase inhibitor (TKI). In various embodiments, the TKI can be Erlotinib, Gefitinib, Apatinib, Cabozantinib, Canertinib, Crenolanib, Damnacanthal, Foretinib, Fostamatinib, Intedanib, Linifanib, Motesanib, Mubritinib, Vatalanib, or Vemurafenib.

In various embodiments, the disease state can be prostate cancer, lung cancer, breast cancer, metastatic cancer, cancer metastasis to the bone, metastatic prostate cancer, metastatic lung cancer or metastatic breast cancer.

Various embodiments of the present invention provide for a method, comprising: obtaining a biological sample from a subject; testing the biological sample for a relative increase, decrease, or steady expression of MEG9; and associating a relative increased expression level of MEG9 with a lower likelihood of drug responsiveness or associating a relative decreased or a steady expression level of MEG9 with a higher likelihood of cancer drug responsiveness.

In various embodiments, the method can further comprise selecting a cancer drug to administer to the subject when a higher likelihood of drug responsiveness. In various embodiments, the drug can be a tyrosine kinase inhibitor (TKI). In various embodiments, the TKI can be Erlotinib, Gefitinib, Apatinib, Cabozantinib, Canertinib, Crenolanib, Damnacanthal, Foretinib, Fostamatinib, Intedanib, Linifanib, Motesanib, Mubritinib, Vatalanib, or Vemurafenib.

Various embodiments provide for a method, comprising: obtaining a biological sample from a subject; testing the biological sample for a relative increase, decrease, or steady expression of MEG9; and associating a relative increased expression the MEG9 with metastatic disease state or associating a relative decreased or a steady expression level of MEG9 with a non-metastatic disease state.

In various embodiments, the disease state can be prostate cancer, lung cancer, breast cancer, metastatic cancer, cancer metastasis to the bone, metastatic prostate cancer, metastatic lung cancer or metastatic breast cancer.

Various embodiments provide for a system, comprising: a biological sample from a subject; and a probe to test the biological sample for a relative increase, decrease, or steady expression of a miRNA. In various embodiments, the system can comprise a machine to associate a relative increase of the miRNA expression level with a lower likelihood of drug responsiveness or associating a relative decrease or a steady expression level of the miRNA with a higher likelihood of cancer drug responsiveness, associate a relative increase of the miRNA expression level with a higher likelihood of having a cancer drug resistant disease state or associating a relative decrease or a steady expression level of the miRNA with cancer drug susceptible disease state, or associate a relative increased expression the DLK1-miRNA with metastatic disease state or associating a relative decreased or a steady expression level of DLK1- miRNA with a non-metastatic disease state.

In various embodiments, the miRNA can be miR-379, miR-379*, miR-193b, miR-193b*, miR-409-5p, miR-409-3p, miR-154, miR-154*, mature miR-379, mature miR-379*, mature miR-193b, mature miR-193b*, mature miR-409-5p, mature miR-409-3p, mature miR-154, mature miR-154*, SEQ ID NO:2, SEQ ID NO:3, SEQ ID NO:5, SEQ ID NO:6, SEQ ID NO:8, SEQ ID NO:9, SEQ ID NO: 11 or SEQ ID NO: 12.

In various embodiments, the cancer or disease state can be prostate cancer, lung cancer, breast cancer, metastatic cancer, cancer metastasis to the bone, metastatic prostate cancer, metastatic lung cancer or metastatic breast cancer.

Various embodiments provide for a system, comprising: a biological sample from a subject; and a probe to test the biological sample for a relative increase, decrease, or steady expression of DLK1-DIO3 cluster/region. In various embodiments, the system can further comprise a machine to associate a relative increased expression level of the DLK1-DIO3 cluster/region with a lower likelihood of drug responsiveness or associating a relative decreased or a steady expression level of the DLK1-DIO3 cluster/region with a higher likelihood of drug responsiveness, associate a relative increased expression level of the DLK1-DIO3 region with a cancer drug resistant disease state or associating a relative decreased or a steady expression level of the DLK1-DIO3 cluster/region with cancer drug susceptible disease state, or associate a relative increased expression the DLK1-DIO3 cluster/region with metastatic disease state or associating a relative decreased or a steady expression level of DLK1-DIO3 cluster/region with a non-metastatic disease state.

In various embodiments, the cancer or disease state can be prostate cancer, lung cancer, breast cancer, metastatic cancer, cancer metastasis to the bone, metastatic prostate cancer, metastatic lung cancer or metastatic breast cancer.

Various embodiments provide for a system, comprising: a biological sample from a subject; and a probe to test the biological sample for a relative increase, decrease, or steady expression of MEG9. In various embodiments, the system can further comprise a machine to associate a relative increased expression level of MEG9 with a lower likelihood of drug responsiveness or associate a relative decreased or a steady expression level of MEG9 with a higher likelihood of cancer drug responsiveness.

Various embodiments provide for a system, comprising: a biological sample from a subject; and a probe to test the biological sample for a relative increase, decrease, or steady expression of MEG9. In various embodiments, the system can further comprise a machine to associate a relative increased expression the MEG9 with metastatic disease state or associate a relative decreased or a steady expression level of MEG9 with a non-metastatic disease state.

In various embodiments, the disease state can be prostate cancer, lung cancer, breast cancer, metastatic cancer, cancer metastasis to the bone, metastatic prostate cancer, metastatic lung cancer or metastatic breast cancer.

Various embodiments provide for a method, comprising: providing a test compound; contacting the test compound with a cell expressing a miRNA; detecting a relative increase, decrease, or steady expression of the miRNA; and identifying the test compound as an inhibitor when a relative decrease of the miRNA expression is detected, or identifying the test compound as an agonist when a relative increase of the miRNA expression is detected.

In various embodiments, miRNA can be miR-379, miR-379*, miR-193b, miR-193b*, miR-409-5p, miR-409-3p, miR-154, miR-154*, mature miR-379, mature miR-379*, mature miR-193b, mature miR-193b*, mature miR-409-5p, mature miR-409-3p, mature miR-154, mature miR-154*, SEQ ID NO:2, SEQ ID NO:3, SEQ ID NO:5, SEQ ID NO:6, SEQ ID NO:8, SEQ ID NO:9, SEQ ID NO: 11 or SEQ ID NO: 12.

Various embodiments provide for a method, comprising: providing a test compound; contacting the test compound with a cell expressing the DLK1-DIO3 region; detecting a relative increase, decrease, or steady expression of the DLK1-DIO3 region; and identifying the test compound as an inhibitor when a relative decrease of the DLK1-DIO3 region expression is detected, or identifying the test compound as an agonist when a relative increase of the DLK1-DIO3 region expression is detected.

Various embodiments provide for a method, comprising: providing a test compound; contacting the test compound with a cell expressing MEG9; detecting a relative increase, decrease, or steady expression of MEG9; and identifying the test compound as an inhibitor when a relative decrease of MEG9 expression is detected, or identifying the test compound as an agonist when a relative increase of MEG9 expression is detected.

Other features and advantages of the invention will become apparent from the following detailed description, taken in conjunction with the accompanying drawings, which illustrate, by way of example, various features of embodiments of the invention.

### BRIEF DESCRIPTION OF THE FIGURES

Exemplary embodiments are illustrated in referenced figures. It is intended that the embodiments and figures disclosed herein are to be considered illustrative rather than restrictive.

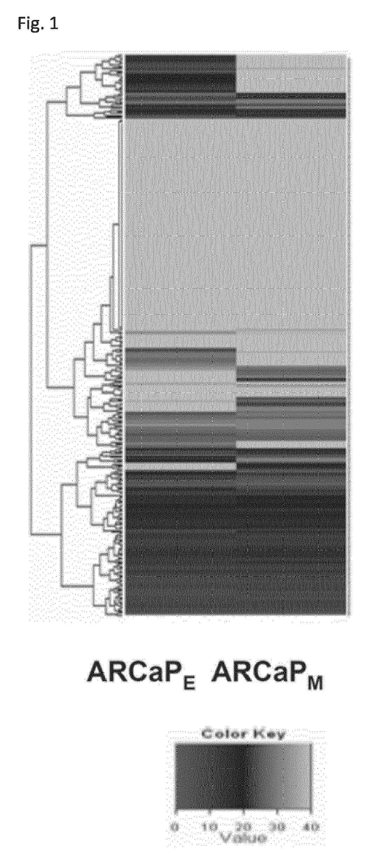

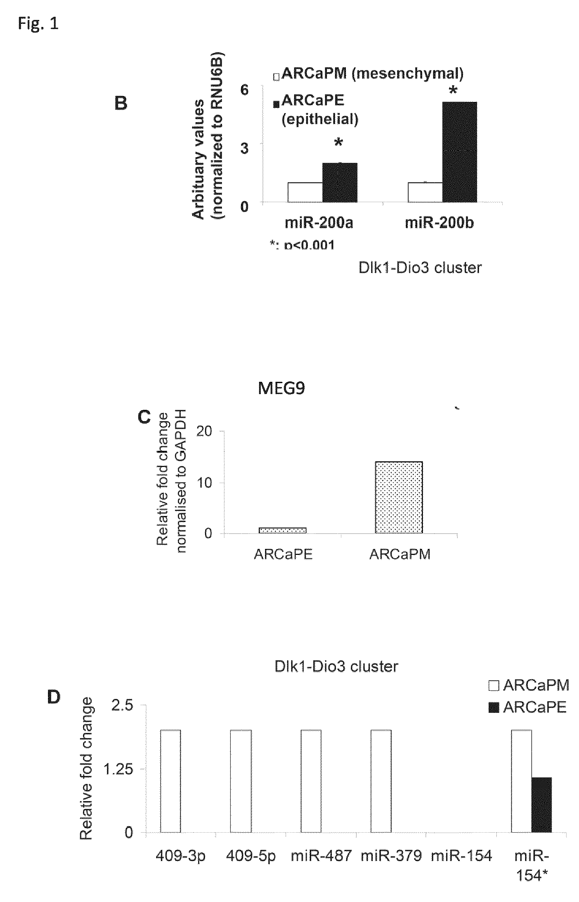

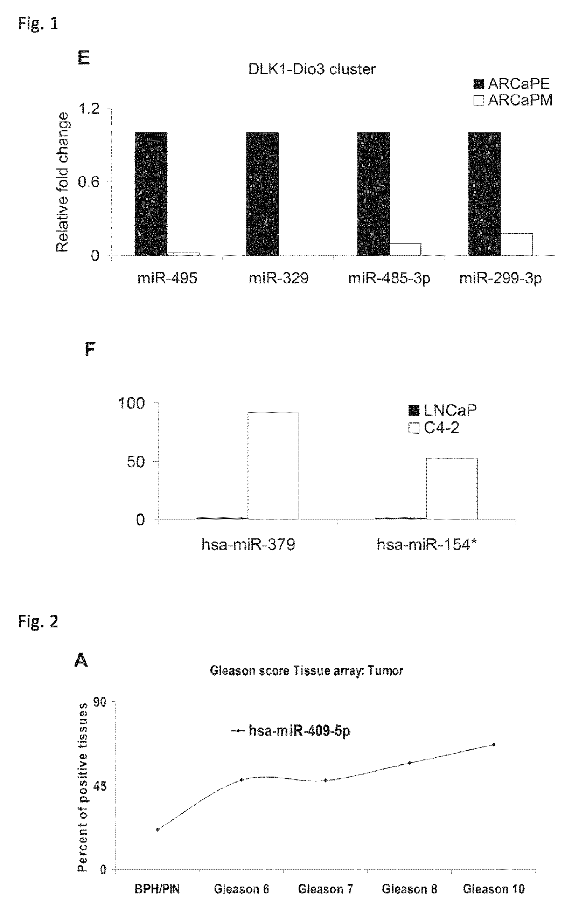

Figure 1 depicts microRNA genomic profiling of prostate cancer bone metastatic models with elevated miRNA in DLK1-DIO3 miRNA cluster in accordance with various embodiments of the present invention. **A.** Heatmap of global miRNA profiling in ARCaP_{E} and ARCaP_{M}. Green indicates low expression, and red indicates high expression. **B.** miR-200 family miRNA expression in ARCaP EMT model. **C.** Expression analysis in long non-coding RNA MEG9 in ARCaP EMT model. **D.** miRNA highly expressed in metastatic mesenchymal cell ARCaP_{M} compared to ARCaP_{E}. **E.** miRNA highly expressed in non-metastatic ARCaP_{E} cells. **F.** miRNA elevated in DLK1-DIO3 cluster in C4-2 prostate cancer cells compared to LNCaP prostate cancer cells.

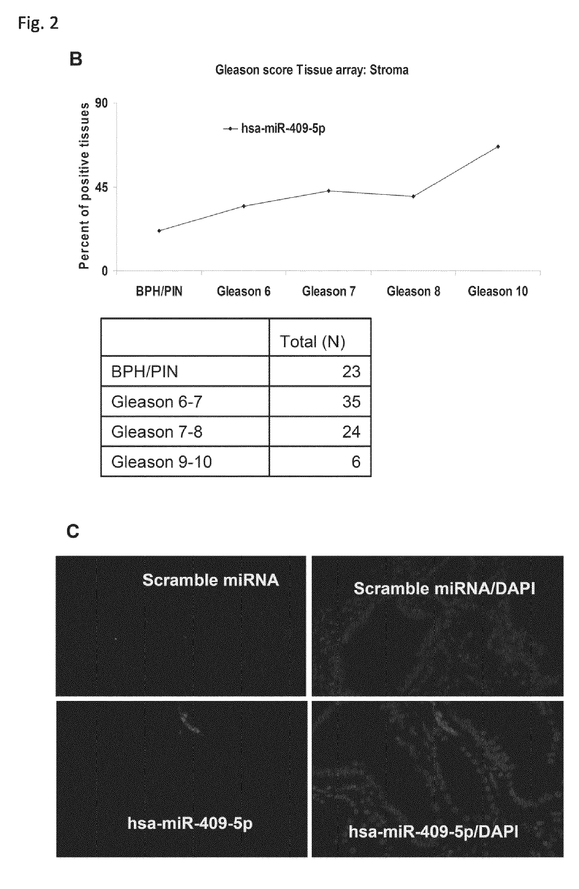

Figure 2 depicts miR-409-5p detection in Gleason score tissue array using *in situ* hybridization and detected by quantum dot analysis in accordance with various embodiments of the present invention. **A.** miR-409-5p expression in tumor cells with Gleason grade. **B.** miR-409-5p expression in stroma cells with Gleason grade. **C.** Representative pictures of miR-409-5p (red) expression in tumor and stroma at 40X. Scramble was used as negative control. DAPI (blue) is used to stain the nucleus.

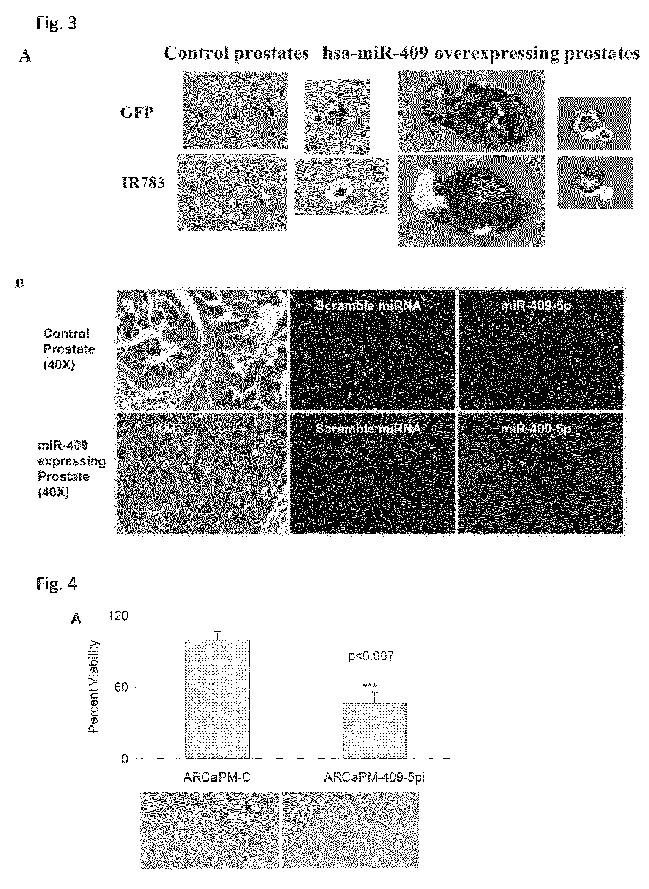

Figure 3 depicts overexpression of miR-409 in prostate gland results in tumor development in accordance with various embodiments of the present invention. **A.** Comparison of normal prostate and miR-409 expressing prostates. Upper panel represent green fluorescence from cells containing green fluorescent protein (GFP) containing control plasmid or miR-409 expressing plasmid. Bottom panel represent tumor specific near infrared dye (IR783) uptake in miR-409 expressing prostates. **B.** H&E staining of normal control prostate and miR-409 expressing prostates (40X), followed by miRNA detection of scramble miRNA and miR-409-5p of control and miR-409 expressing tissues by *in situ* hybridization and quantum dots detection (40X).

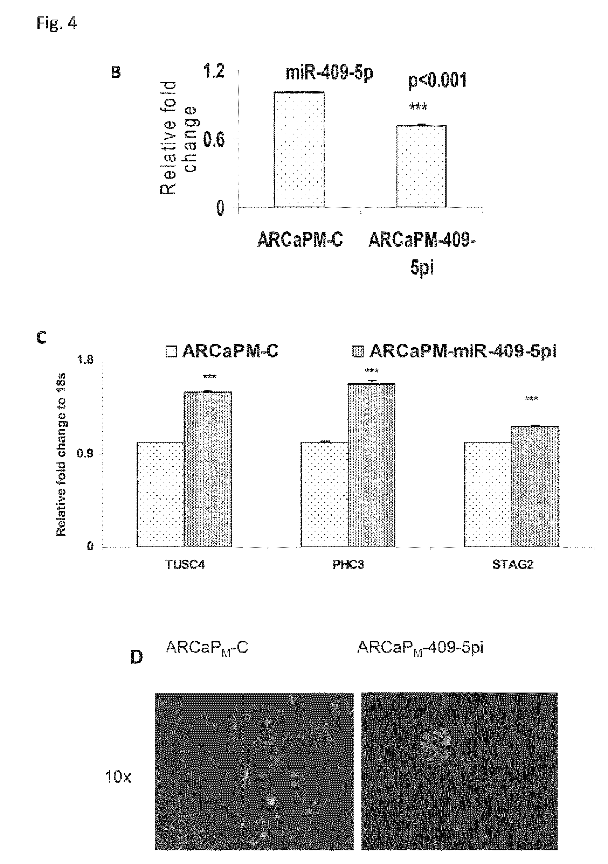

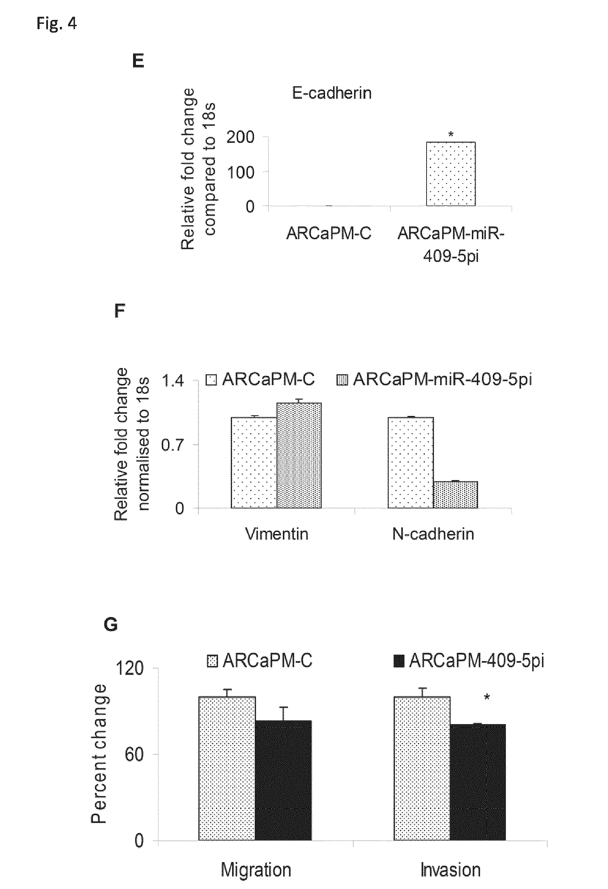

Figure 4 depicts inhibition of miR-409-5p results in reversion of EMT. **A*.*** Percent viability of ARCaP_{M}-C and ARCaP_{M}-409-5p cells after transient transfection of miR-409-5p inhibitor. **B.** Expression of miR-409-5p assayed by real time PCR in ARCaP_{M} prostate cancer cells with miR-409-5p inhibitor normalized to RNU6B. **C.** RNA expression of miR-409-5p targets in ARCaP_{M} prostate cancer cells assayed by real time PCR. miR-409-5p mRNA targets: TUSC4, PHC3, and STAG2. **D.** Morphological EMT changes in miR-409-5p inhibited ARCaP_{M} cells; magnification 10x. **E.** and **F.** RNA expression assayed by real time PCR of EMT markers, E-cadherin, vimentin and N-cadherin. **G.** Migration and Invasion assay of ARCaP_{M}-C and ARCaP_{M}-409-5pi cells.

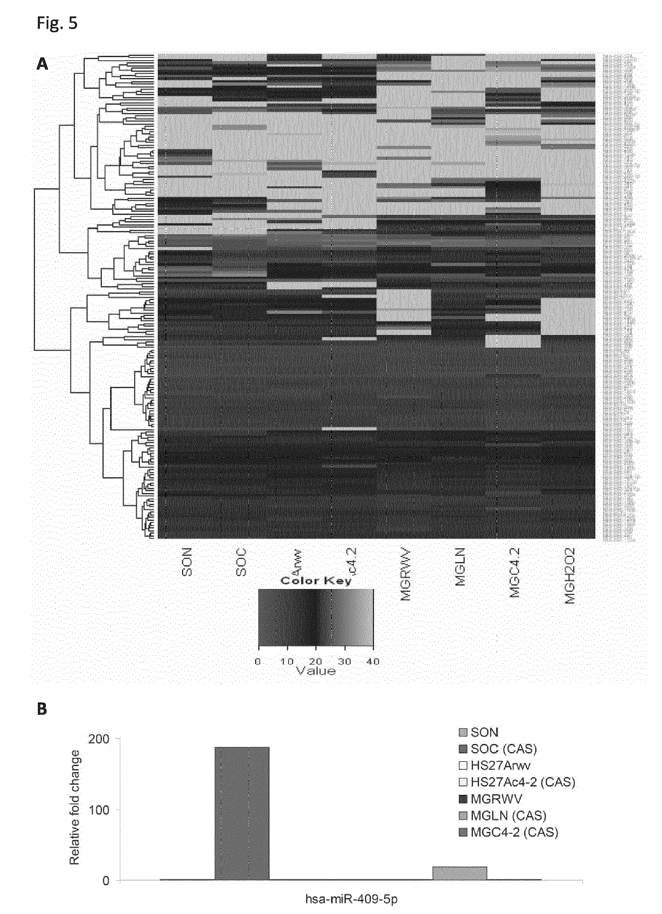

Figure 5 depicts microRNA genomic profiling of normal and cancer associated stroma from patients and 3-dimensional cell models in accordance with various embodiments of the present invention. **A.** i. Heatmap of global miRNA profiling in prostate and bone normal fibroblasts and cancer associated fibroblasts. Numbers represent Ct values, where green indicates low expression, and red indicated high expression. SON (patient normal prostate fibroblasts), SOC (patient prostate cancer associated fibroblast), HS-27a_{RWV} and MG_{RWV} are bone stromal cells, HS-27a_{C4-2}, MG_{LN} and MG_{C4-2} are bone cancer associated stromal cells derived by 3D culture. MG_{H2O2} is MG-63 fibroblast exposed to hydrogen peroxide. **ii.** Expression levels of miR-409-Sp in normal and cancer associated stroma.

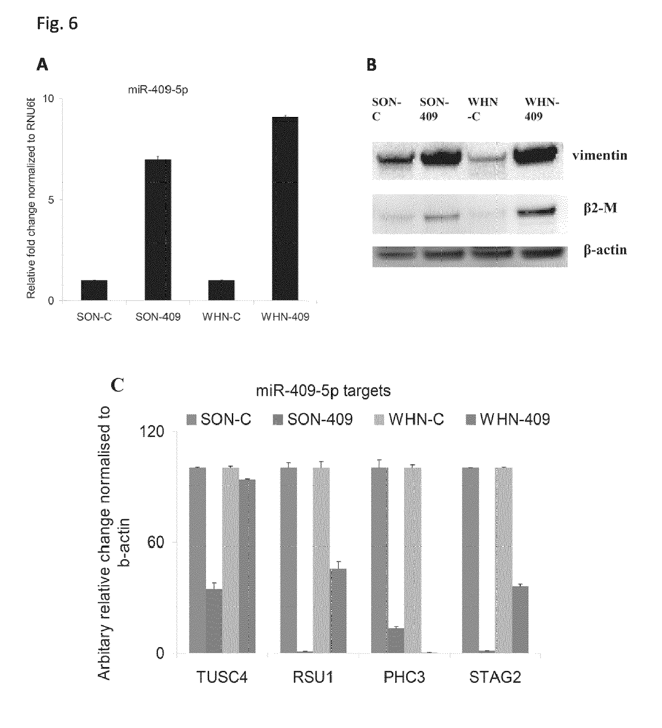

Figure 6 shows that overexpression of miR-409 in normal stromal cells results in activated fibroblasts in accordance with various embodiments of the present invention. **A.** miRNA expression levels of miR-409 in normal prostatic stromal fibroblasts, SON-C and WHN-C and miR-409 expressing cells SON-409 and WHN-409. **B.** Protein expression levels of vimentin, β2-M and β-actin in normal prostatic stroma and miR-409 overexpressing stroma. **C.** Target mRNA of miR-409-5p in prostatic stromal cells. miR-409-5p targets: PHC3 (Polyhomeotic protein 3), TUSC4 (tumor suppressor 1), RSU1 (Ras suppressor protein 1) and STAG2 (Stromal antigen 1) which have 8mer match seed sequence from TargetScan.

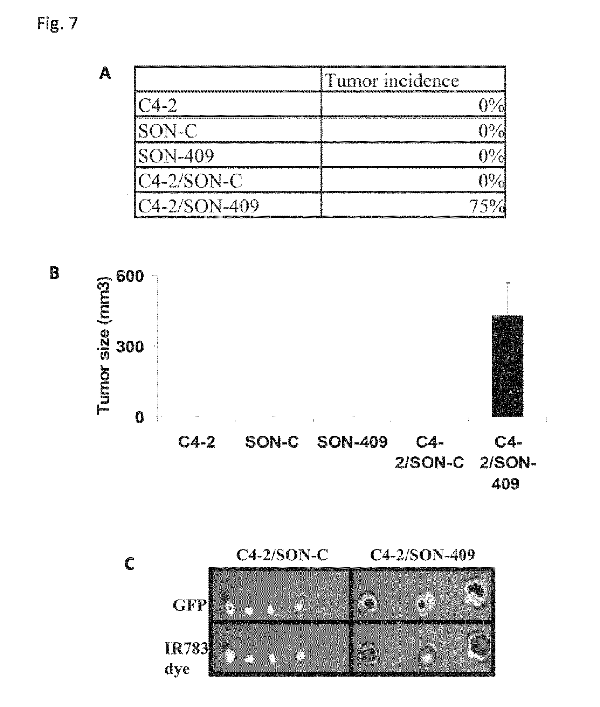

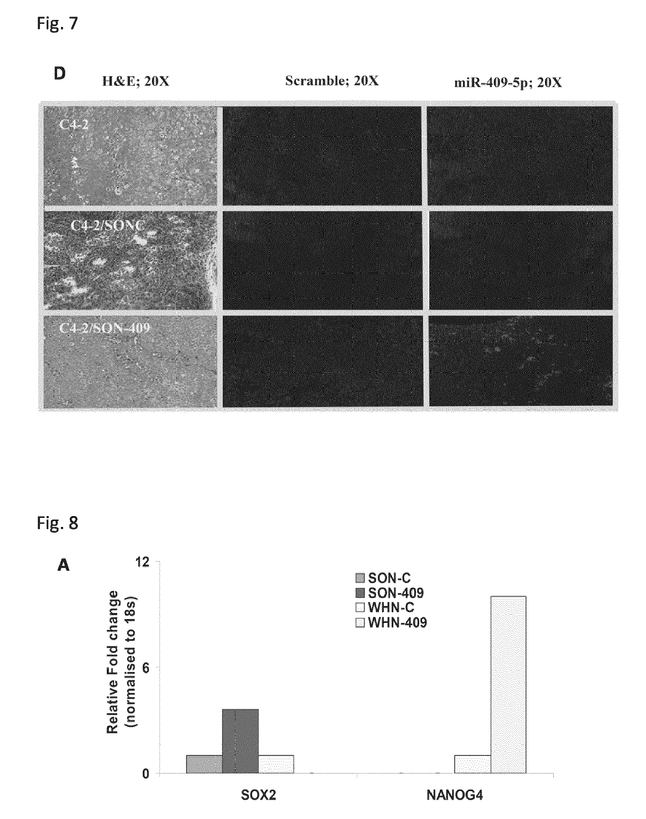

Figure 7 shows that overexpression of miR-409 in normal stroma cells plays an inductive role in prostate cancer tumor growth in accordance with various embodiments of the present invention. **A.** Tumor incidence comparing different groups at 5 weeks after subcutaneous tumor injection in nude mice. Groups: C4-2 (prostate cancer cell line), SON-C (normal prostatic stromal cells transfected with control plasmid), SON-409 (normal prostatic stromal cells transfected with miR-409 overexpression plasmid), C4-2/SON-C (combination of cancer and normal prostate stromal cells), C4-2/SON-409 (combination of prostate cancer and prostate stromal cells with miR-409 overexpression). **B.** Tumor size of different groups at 5 weeks after tumor injection. **C.** Images of tumor using green fluorescence imaging for stromal cells and near infrared imaging for tumor cells in C4-2/SON-C and C4-2/SON-409 tumors. **D.** *In situ* hybridization using scramble and hsa-miR-409-5p miRNA probe on C4-2/SON-C tumors and C4-2/SON-409 tumors. Quantum dots imaging of miRNA. Blue: DAPI stain for nucleus, Red: hsa-miR-409-biotin probe.

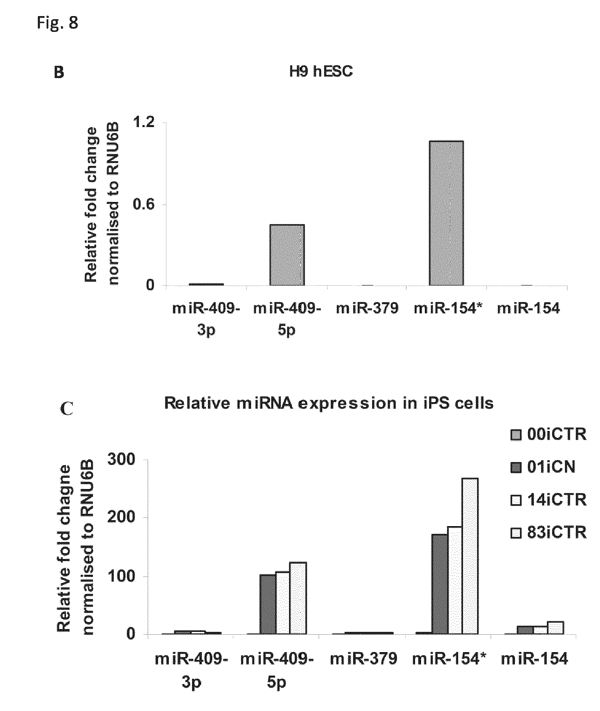

Figure 8 depicts miR-409-5p elevated in human embryonic stem cells and human induced pluripotent stem cells. **A.** miRNA expression of miR-409 cluster members in human embryonic stem cells. **B.** miRNA expression of miR-409 cluster in induced pluripotent stem cells. **C.** mRNA expression analysis of stem cell markers in normal and miR-409 overexpressing prostatic stromal cells.

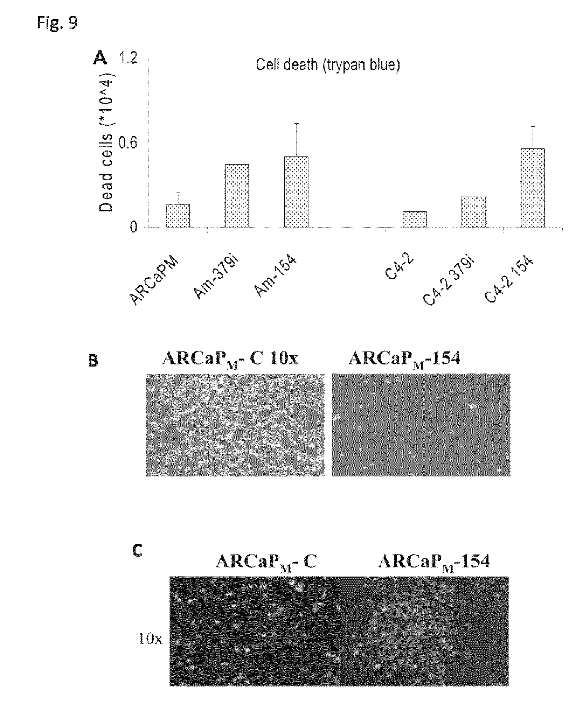

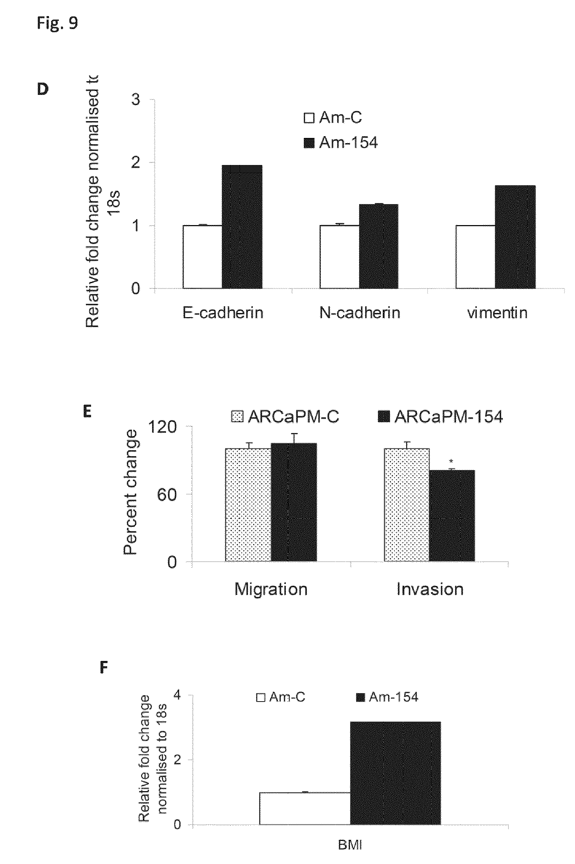

Figure 9 depicts inhibition of miR-154* and miR-379 induces cell death and EMT in aggressive prostate cancer cells. **A.** Cell death assayed by trypan blue assay in ARCaP_{M} and C4-2 cells transfected with control, miR-154* and miR-379 inhibitor by lentiviral transduction. **B.** Images of ARCaP_{M} cells transfected with control and miR-154* inhibitor. **C.** MET in ARCaP_{M} cells transfected with miR-154 and miR-379 inhibitor. **D.** mRNA expression of E-cadherin, N-cadherin and vimentin in ARCAPm-C and Am-154. **E.** Migration and invasion in Am-C and AM-154. **F.** miR-154* targets in Am-C and Am-154 cells.

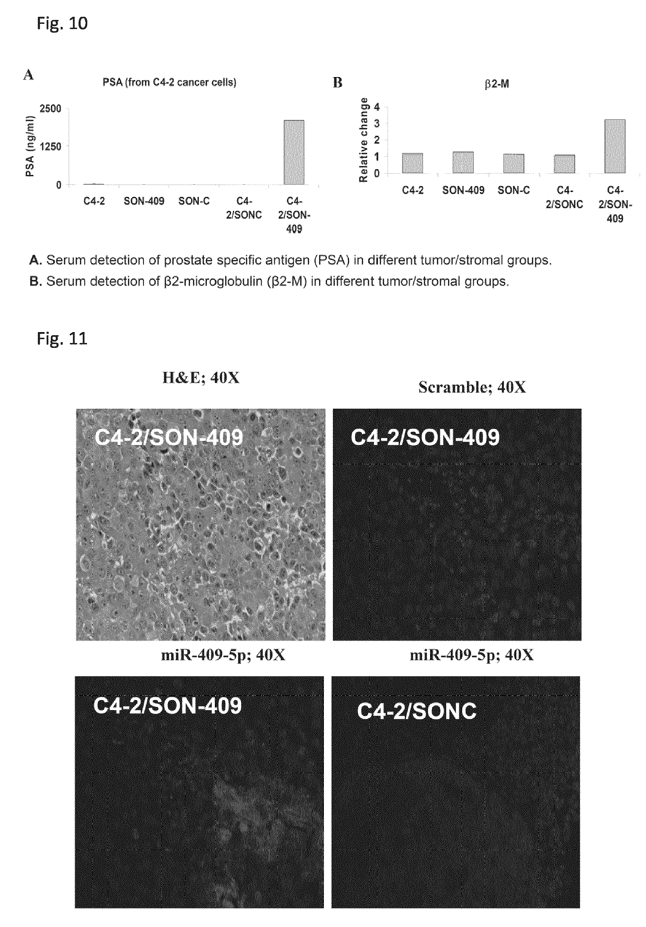

Figure 10 depicts serum detection of prostate specific antigen (PSA) in different tumor/stromal groups, and serum detection of β2- microglobulin (β2-M) in different tumor/stromal groups in accordance with various embodiments of the present invention.
Figure 11 depicts H&E staining and miRNA detection in accordance with various embodiments of the present invention.

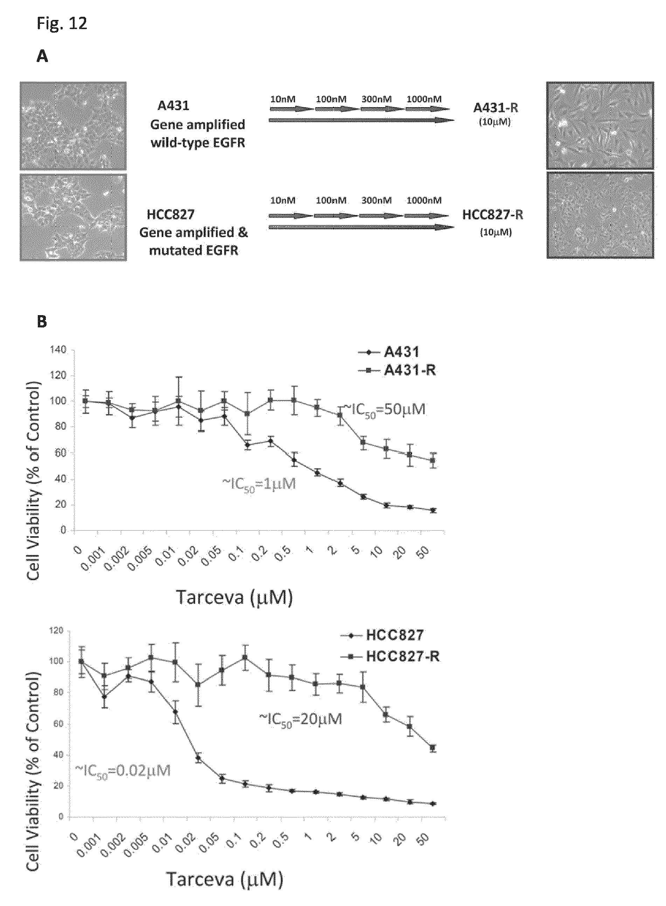

Figure 12 depicts the generation and demonstration of EGFR-TKI acquired resistance in two independent lung cancer models in accordance with various embodiments of the present invention. Two cell lines, A431 and HCC827, which are representative of NSCLC patients that acquire resistance to EGFR-TKIs were chosen. A431 has a gene-amplified EGFR whereas HCC827 has a gene-amplified EGFR with kinase domain somatic mutations. Both cell lines are highly sensitive to EGFR TKIs. The cell lines were progressively treated with increasing doses (up to 10mM) of EGFR-TKI, TARCEVA, until they acquired resistance to the drug (A431-R and HCC827-R) as shown in a cell viability assay.

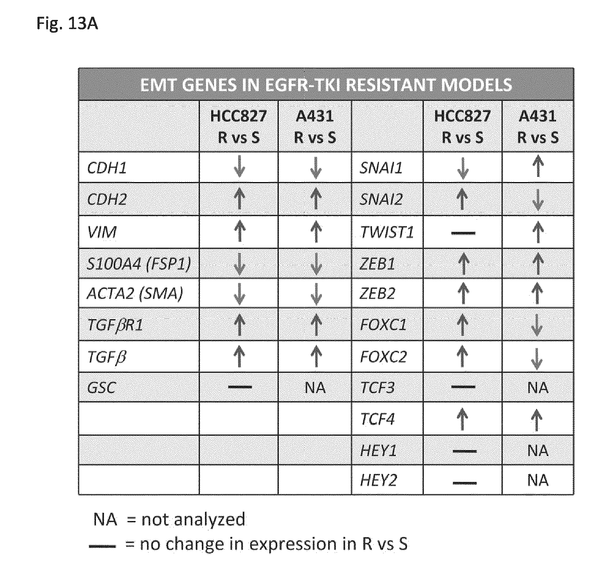

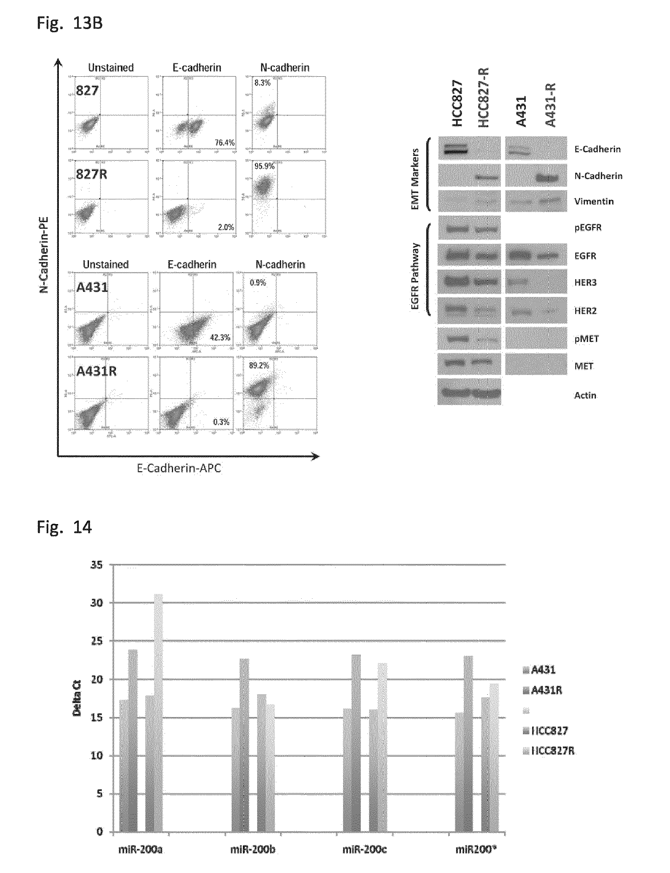

Figure 13 depicts EGFR-TKI acquired resistance results in epithelial to mesenchymal transition (EMT) in accordance with various embodiments of the present invention. It is demonstrated herein that the NSCLC cells that acquire resistance to EGFR-TKI undergo the transdifferentiation process of EMT. EMT markers N-cadherin and Vimentin are higher in mesenchymal cells whereas E-cadherin is higher in epithelial cells. EMT transcription factors SNAI1, SNAI2, TWIST1, ZEB1, ZEB2 are typically higher in mesenchymal cells. Figure 13a summarizes the RNA analysis data for EMT genes, E-cadherin (CDH1), N-cadherin (CDH2), Vimentin (VIM), FSP1, SMA, TGbR1, TGFb and EMT transcription factors, SNAI1, SNAI2, TWIST1, ZEB1, ZEB2, FOXC1, FOXC2, TCF3, TCF4. ZEB1, ZEB2 and TCF4 are higher in resistant cells that are of the mesenchymal phenotype. Figure 13b shows the decrease in E-cadherin and increase in N-cadherin proteins in a western blot and flow cytometry analysis in the resistance models. These changes are indicative of an EMT phenomenon.
Figure 14 shows that members of the miR-200 family decrease with acquisition of EGFR-TKI resistance in accordance with various embodiments of the present invention. A total of 330 microRNAs (miRs) were screened to identify miRs that are modulated with EMT. 29 of these miRs were tested in NSCLC EGFR-TKI acquired resistance model systems to evaluate which of these miRs are also differentially expressed with EGFR-TKI acquired resistance. miR-200 family members inhibit the EMT transcription factors, ZEB1 and ZEB2, that repress E-cadherin. They are expected to be higher in epithelial cells where expression of ZEB1 and ZEB2 is lower as compared to their mesenchymal counterparts. The miR expression in both the resistant models as compared to their drug sensitive counterparts is shown and is represented as delta Ct. Higher value of delta Ct represents lower expression where 1 Ct value equals 2-fold expression. miR-200a and miR-200c are downregulated in HCC-827R mesenchymal cells whereas miR-200a, miR-200b, miR-200c and miR-200* are downregulated in A431R mesenchymal cells.

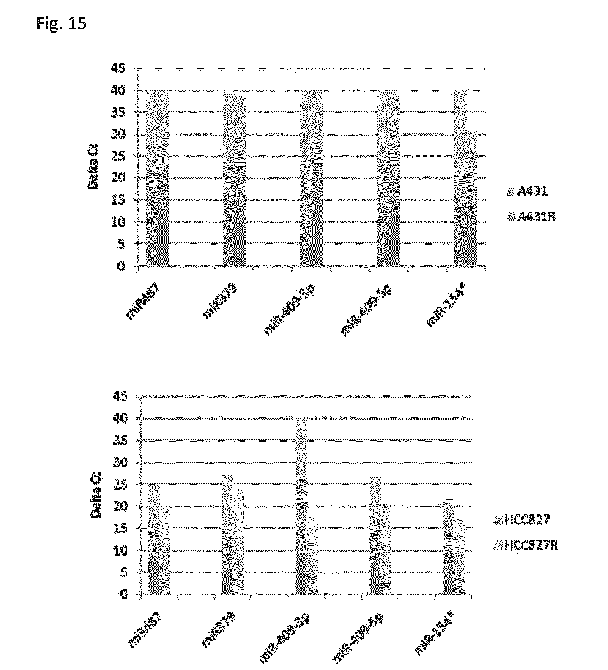

Figure 15 shows that members of the miR-379 and miR-154 family increase with acquisition of EGFR-TKI resistance in accordance with various embodiments of the present invention. Members of the miR-379 family (miR-379) and miR-154 family (miR-487, miR-409-3p, miR-409-5p and miR-154*) are upregulated in the EGFR-TKI resistant mesenchymal NSCLC cells. In A431R, an increase in miR-379 (~4-fold) and miR-154* (~20-fold) as compared to A431 are observed. In HCC-827R, an increase in miR-379 (~6-fold), miR-487 (~8-9 fold), miR-409-3p (>46-fold), miR-409-5p (~14-fold) and miR-154* (~8-fold) as compared to HCC827 cells are observed.

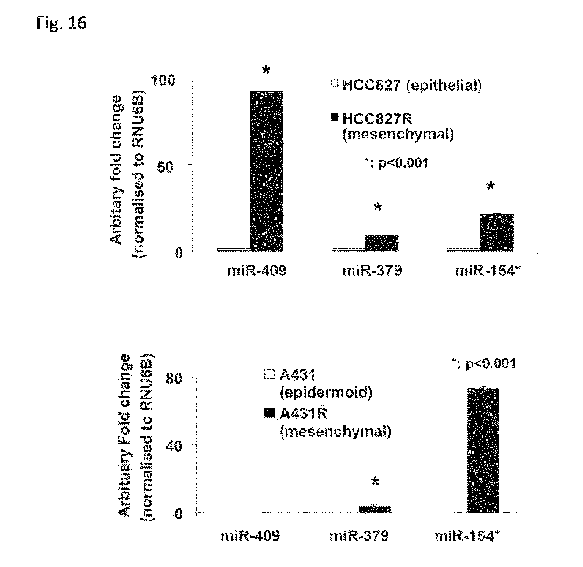

Figure 16 shows that increase in members of the miR-379 and miR-154 family is seen in the intermediate generations of drug resistance acquisition in accordance with various embodiments of the present invention. As the EGFR-TKI drug resistance was progressively acquired as the drug concentration was increased, the intermediate cell populations generated during the drug resistance process were examined. A marker of drug resistance should be concordant with acquisition of the drug resistance and should increase as the resistance gets stronger. As shown in Fig. 5, miRs -379, 409-3p, 409-5p and 154* increase with intermediate EGFR-TKI resistant -TKI populations (drug concentration of the intermediate population is shown) with the highest expression in HCC827R (generated at 10mM EGFR-TKI).

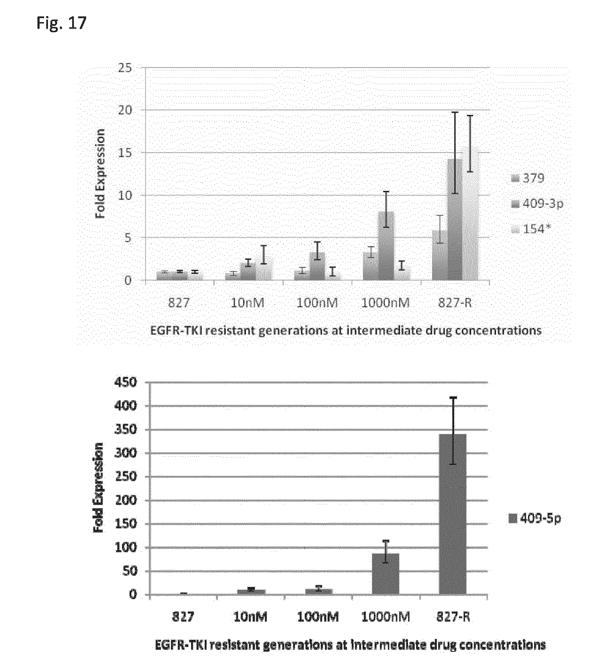

Figure 17 depicts evidence in literature indicates that members of miR-379 and miR-154 family are increased in pluripotent stem cells in accordance with various embodiments of the present invention. There is also evidence demonstrating the presence of miR-154 family members in fetal lung which is lost in the adult lung.

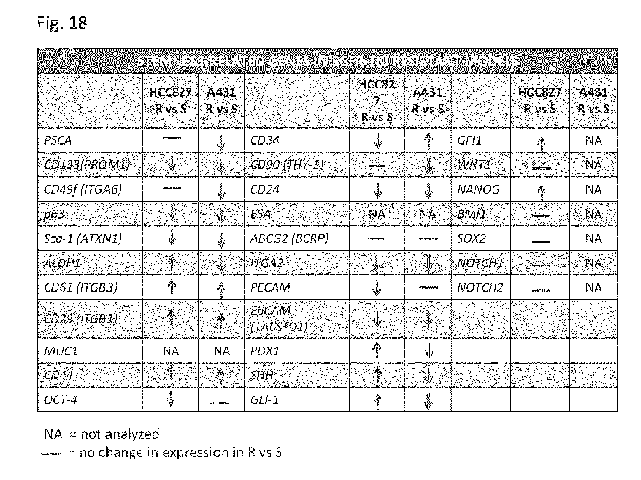

Figure 18 depicts a summary of sternness-related genes in EGFR-TKI resistant NSCLC models in accordance with various embodiments of the present invention. A panel of genes that have been suggested as cancer stem cell genes in other systems were interrogated in EGFR-TKI resistant systems and are their expression summary is shown. A higher number of stemness related genes are upregulated in HCC827R as compared to the A431R systems.

### DESCRIPTION OF THE INVENTION

All references cited herein are incorporated by reference in their entirety as though fully set forth. Unless defined otherwise, technical and scientific terms used herein have the same meaning as commonly understood by one of ordinary skill in the art to which this invention belongs. Singleton et al., Dictionaiy of Microbiology and Molecular Biology 3rd ed., J. Wiley & Sons (New York, NY 2001); March, Advanced Organic Chemistry Reactions, Mechanisms and Structure 5th ed., J. Wiley & Sons (New York, NY 2001); and Sambrook and Russel, Molecular Cloning: A Laboratory Manual 3rd ed., Cold Spring Harbor Laboratory Press (Cold Spring Harbor, NY 2001), provide one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described. For purposes of the present invention, the following terms are defined below.

"Cancer" and "cancerous" refer to or describe the physiological condition in mammals that is typically characterized by unregulated cell growth. Examples of cancer include, but are not limited to, lung cancer, prostate cancer, breast cancer, colon cancer, hepatocellular cancer, gastric cancer, pancreatic cancer, cervical cancer, ovarian cancer, liver cancer, bladder cancer, cancer of the urinary tract, thyroid cancer, renal cancer, carcinoma, melanoma, head and neck cancer, and brain cancer.

"Mammal" as used herein refers to any member of the class Mammalia, including, without limitation, humans and nonhuman primates such as chimpanzees, and other apes and monkey species; farm animals such as cattle, sheep, pigs, goats and horses; domestic mammals such as dogs and cats; laboratory animals including rodents such as mice, rats and guinea pigs, and the like. The term does not denote a particular age or sex. Thus adult and newborn subjects, as well as fetuses, whether male or female, are intended to be including within the scope of this term.

"Treatment" and "treating," as used herein refer to both therapeutic treatment and prophylactic or preventative measures, wherein the object is to prevent, slow down and/or lessen the disease even if the treatment is ultimately unsuccessful.

MicroRNAs were demonstrated to be oncogenic in a variety of cancers, as described herein, the inventors profiled microRNA expression in multiple human bone metastatic prostate cancer cell lines and cancer associated stroma of the prostate and the bone. MiR-409-5p, miR-379 and miR-154* are highly expressed in both the cancer cells and the adjacent stroma and microRNA miR-409-5p is oncogenic *in vivo,* plays an important role in EMT and targeting miR-409-5p, miR-379 and miR-154* results in cell death of prostate cancer cells.

One of the largest microRNA clusters is on human chromosome 14q32. Its orthologous region in mouse is situated on the long arm of chromosome 12. About 10% of the microRNAs currently known in mouse and human are located in this cluster. This cluster is located within a well-known imprinted region that is characterized by parental-origin-specific mono-allelic expression of the encompassed genes (genomic imprinting is an epigenetically heritable mechanism where maternal or paternal alleles are methylated). In this study, the inventors demonstrate that microRNA members of the DLK-DIO3 cluster located in human chromosome including microRNA miR-409-5p, miR-379 and miR-154* are highly expressed in bone metastatic mesenchymal type prostate cancer cell lines. Overexpression of miR-409 in normal prostate resulted in tumor development in mice and tumors that developed varied from benign hyperplasia to adenocarcinoma. More importantly, miR-409-5p expression was upregulated in high Gleason score human prostate cancer tissue array. Inhibition of miR-409-5p, miR-379 and miR-154* either alone or blocking all three microRNAs together using a siRNA based approach resulted in increased cell death, reversal of epithelial to mesenchymal transition (EMT), biochemically and functionally. In addition to cancer cells, miR-409-5p was markedly upregulated in cancer associated stroma derived from prostate and the bone compared to normal stroma. Ectopic expression of miR-409 in normal stroma led to conversion of the stroma to a cancer associated stroma and miR-409 expressing stroma co-injected with less aggressive cancer cells had explosive tumor growth in vivo. This is the first demonstrated evidence of an oncogenic microRNA in prostate cancer *in vivo.* Thus, miR-409-5p appears to be a promising therapeutic target to inhibit the vicious cycle involving bi-directional tumor stromal interactions in prostate cancer.

The latest development in the inventors' understanding of tumor microenvironment has provided a new opportunity for a fundamental change of approaches to cancer drug discovery. In addition to targeting the cancer cells, there is a need to focus on new molecular targets and pathways essential for the cells surrounding the cancer cells including stromal cells that have been demonstrated by recent studies to promote cancer growth. Impairment of stroma cell function in the cancer microenvironment is believed to be an important step in tumor progression. In addition, co-targeting of stromal cells in addition to cancer cells will lead to better killing of cancer cells. It has been demonstrated that fibromuscular stroma and stromal fibroblasts play regulatory role in prostate development and prostate carcinogenesis. In these studies, urogenital sinus mesenchyme (UGM) or embryonic/adult stromal fibroblasts were shown to drive the growth of UG epithelium and prostate cancer (25). These studies for the first time suggested that androgen receptor signaling from the stroma is critical for the development and differentiation of the normal prostate epithelium (25,26). Using cell recombination studies, the progression of prostate cancer from androgen-dependent to androgen-independent states and the subsequent progression to bone metastatic phenotypes can be achieved by cellular interactions between prostate cancer and prostate or bone stromal cells in mice *in vivo* or when co-cultured under three-dimensional (3D) conditions (27). It has been established that the fibroblasts adjacent to the cancer tissue or cancer-associated fibroblasts (CAF) are structurally and functionally different from fibroblasts adjacent to normal epithelium. These cells exhibit marked differences in gene expression profiles and have been shown to predict the progression of prostate cancer (28). The inventors demonstrated previously that the reciprocal cellular interaction between prostate cancer and CAF or stromal fibroblasts from different zonal origin (27). These findings, taken together, emphasized the important role of the stromal and tumor microenvironment in prostate cancer progression (16,29-31). These studies highlight the bidirectional interactions and co-evolution of tumor-stroma in cancer progression (32). Therapies that target many of the stromal factors have been successfully tested in preclinical models and/or in clinical trials to treat prostate cancer and other solid tumors.

EMT is a highly conserved process where polarized immotile epithelial cells transition to motile mesenchymal cells. EMT is commonly associated with cancer migration, invasion and metastasis. The common feature of EMT is the loss of E-cadherin and an increase in vimentin. In cancer, EMT facilitates benign tumors to infiltrate surrounding tissues and metastasize to soft tissues and the bone. In prostate cancer, EMT has been described in the androgen refractory prostate cancer (ARCaP) cell model (3,17,23,28,33). Prostate cancer cell lines and clinical samples are shown to express RANKL and secrete soluble factors such as β2M, which is not only responsible for driving EMT and bone metastasis of human prostate cancer cells but also exerted the same effects by promoting EMT and bone metastasis in human breast, renal and lung cancer cells. The resulting ARCaP_{M} cells had high levels of the mesenchymal markers such as vimentin, N-cadherin and Snail and exhibit 100% incidence of bone metastasis when injected intracardially.

In conclusion, the inventors provide evidence that oncogenic microRNA miR-409-5p mediated regulation of gene expression in fibroblasts differentially affects epithelial growth and oncogenesis. Previous studies have indicated that secretory factors like TGF-β and its signaling through its receptor in stromal cells influence the carcinogenesis process in adjacent epithelia. The inventors' study defines the role of posttranscriptional regulators of gene expression (microRNAs) can suppress tumor suppressor genes and activate pleiotropic growth factors like beta2-microglobulin in stromal cells and thus can have an effect on adjacent epithelial cells in vivo. The phenotype of the cancer ranges from prostate intraepithelial neoplasia to adenocarcinoma. Strikingly, miR-409-5p is also expressed by metastatic prostate cancer cells and its inhibition leads to cell death. In addition to miR-409-5p, targeting other members of DLK-DIO3 cluster including miR-379 and miR-154* appears to be a promising therapeutic target to inhibit the vicious cycle involving bi-directional tumor stromal interactions in prostate cancer.

Various embodiments of the present invention are based, at least in part, on the inventors' findings described herein.

### Treatments

Various embodiments of the present invention provide for a method of treating cancer in a subject in need thereof, comprising providing a miRNA inhibitor and administering the miRNA inhibitor to the subject.

Various embodiments of the present invention provide for a method of treating cancer metastasis in a subject in need thereof, comprising providing a miRNA inhibitor and administering the miRNA inhibitor to the subject.

Various embodiments of the present invention provide for a method of lowering or treating cancer drug resistance in a subject in need thereof, comprising providing a miRNA inhibitor and administering the miRNA inhibitor to the subject.

Various embodiments of the present invention provide for a method of treating cancer in a subject in need thereof, comprising providing a miRNA inhibitor and administering the miRNA inhibitor to the subject in combination with radiation treatment or chemotherapy treatment.

In certain embodiments, the cancer is prostate cancer. In certain embodiments, the cancer is lung cancer. In certain embodiments, the cancer is breast cancer. In certain embodiments, the cancer is metastatic cancer. In certain embodiments, the metastatic cancer is metastasis to the bone. In certain embodiments, the cancer is metastatic prostate cancer. In certain embodiments, the cancer is metastatic lung cancer. In certain embodiments, the cancer is metastatic breast cancer.

In various embodiments, the miRNA inhibitor is capable of inhibiting miR-379, miR-379*, miR-193b, miR-193b*, miR-409-5p, miR-409-3p, miR-154, and/or miR-154*. In certain embodiments the miRNA inhibitor is capable of inhibiting mature miR-379, miR-379*, miR-193b, miR-193b*, miR-409-5p, miR-409-3p, miR-154, and/or miR-154*. In particular embodiments, the miRNA inhibitor is capable of inhibiting mature miR-379, miR-193b, miR-409-5p, miR-409-3p or miR-154*.

In certain embodiments, the miRNA inhibitor is a shRNA or a siRNA capable of interfering with the expression of SEQ ID NO:2, SEQ ID NO:3, SEQ ID NO:5, SEQ ID NO:6, SEQ ID NO:8, SEQ ID NO:9, SEQ ID NO: 11 or SEQ ID NO: 12. In particular embodiments, the miRNA inhibitor is a shRNA or a siRNA capable of interfering the expression of SEQ ID NO:2, SEQ ID NO:5, SEQ ID NO: 8, SEQ ID NO:9, or SEQ ID NO:12.

In certain embodiments, the miRNA inhibitor is a short hairpin RNA (shRNA) directed against mature miRNAs. In certain embodiments, the shRNA is encoded by a polynucleotide as disclosed by SEQ ID NO:1, SEQ ID NO:4, SEQ ID NO:7, or SEQ ID NO:10. A composition comprising a polynucleotide comprising or as disclosed by SEQ ID NO:1, SEQ ID NO:4, SEQ ID NO:7, or SEQ ID NO:10, for example, as a plasmid, can be administered to the subject. Thereafter, the shRNA is expressed *in vivo* after the administration of the polynucleotide, and inhibits its target miRNA sequence.

In certain embodiments, the miRNA inhibitor is a siRNA directed against mature miRNAs. In various embodiments, the siRNA is as disclosed by SEQ ID NO:3 (to target and inhibit miRNA379), SEQ ID NO:6 (to target and inhibit miRNA193b), or SEQ ID NO:11 (to target and inhibit miRNA154*).

In various embodiments, the miRNA inhibitor is a morpholino antisense oligonucleotide capable of interfering with the expression of SEQ ID NO:2, SEQ ID NO:3, SEQ ID NO:5, SEQ ID NO:6, SEQ ID NO:8, SEQ ID NO:9, SEQ ID NO: 11 or SEQ ID NO: 12. In particular embodiments, the miRNA inhibitor is a morpholino antisense oligonucleotide capable of interfering with the expression of SEQ ID NO:2, SEQ ID NO:5, SEQ ID NO: 8, SEQ ID NO:9, or SEQ ID NO: 12. In various embodiments, the morpholino antisense oligonucleotide is as disclosed by SEQ ID NO:13, SEQ ID NO: 14, or SEQ ID NO: 15.

In various embodiments, the present invention provides pharmaceutical compositions including a pharmaceutically acceptable excipient along with a therapeutically effective amount of a miRNA inhibitor. "Pharmaceutically acceptable excipient" means an excipient that is useful in preparing a pharmaceutical composition that is generally safe, non-toxic, and desirable, and includes excipients that are acceptable for veterinary use as well as for human pharmaceutical use. Such excipients may be solid, liquid, semisolid, or, in the case of an aerosol composition, gaseous.

In various embodiments, the pharmaceutical compositions according to the invention may be formulated for delivery via any route of administration. "Route of administration" may refer to any administration pathway known in the art, including but not limited to aerosol, nasal, oral, transmucosal, transdermal or parenteral. "Transdermal" administration may be accomplished using a topical cream or ointment or by means of a transdermal patch.

"Parenteral" refers to a route of administration that is generally associated with injection, including intraorbital, infusion, intraarterial, intracapsular, intracardiac, intradermal, intramuscular, intraperitoneal, intrapulmonary, intraspinal, intrasternal, intrathecal, intrauterine, intravenous, subarachnoid, subcapsular, subcutaneous, transmucosal, or transtracheal. Via the parenteral route, the compositions may be in the form of solutions or suspensions for infusion or for injection, or as lyophilized powders.

Via the enteral route, the pharmaceutical compositions can be in the form of tablets, gel capsules, sugar-coated tablets, syrups, suspensions, solutions, powders, granules, emulsions, microspheres or nanospheres or lipid vesicles or polymer vesicles allowing controlled release. Via the parenteral route, the compositions may be in the form of solutions or suspensions for infusion or for injection.

Via the topical route, the pharmaceutical compositions based on compounds according to the invention may be formulated for treating the skin and mucous membranes and are in the form of ointments, creams, milks, salves, powders, impregnated pads, solutions, gels, sprays, lotions or suspensions. They can also be in the form of microspheres or nanospheres or lipid vesicles or polymer vesicles or polymer patches and hydrogels allowing controlled release. These topical-route compositions can be either in anhydrous form or in aqueous form depending on the clinical indication. Via the ocular route, they may be in the form of eye drops.

The pharmaceutical compositions according to the invention can also contain any pharmaceutically acceptable carrier. "Pharmaceutically acceptable carrier" as used herein refers to a pharmaceutically acceptable material, composition, or vehicle that is involved in carrying or transporting a compound of interest from one tissue, organ, or portion of the body to another tissue, organ, or portion of the body. For example, the carrier may be a liquid or solid filler, diluent, excipient, solvent, or encapsulating material, or a combination thereof. Each component of the carrier must be "pharmaceutically acceptable" in that it must be compatible with the other ingredients of the formulation. It must also be suitable for use in contact with any tissues or organs with which it may come in contact, meaning that it must not carry a risk of toxicity, irritation, allergic response, immunogenicity, or any other complication that excessively outweighs its therapeutic benefits.

The pharmaceutical compositions according to the invention can also be encapsulated, tableted or prepared in an emulsion or syrup for oral administration. Pharmaceutically acceptable solid or liquid carriers may be added to enhance or stabilize the composition, or to facilitate preparation of the composition. Liquid carriers include syrup, peanut oil, olive oil, glycerin, saline, alcohols and water. Solid carriers include starch, lactose, calcium sulfate, dihydrate, terra alba, magnesium stearate or stearic acid, talc, pectin, acacia, agar or gelatin. The carrier may also include a sustained release material such as glyceryl monostearate or glyceryl distearate, alone or with a wax.

The pharmaceutical preparations are made following the conventional techniques of pharmacy involving milling, mixing, granulation, and compressing, when necessary, for tablet forms; or milling, mixing and filling for hard gelatin capsule forms. When a liquid carrier is used, the preparation will be in the form of a syrup, elixir, emulsion or an aqueous or non-aqueous suspension. Such a liquid formulation may be administered directly p.o. or filled into a soft gelatin capsule.

The pharmaceutical compositions according to the invention may be delivered in a therapeutically effective amount. The precise therapeutically effective amount is that amount of the composition that will yield the most effective results in terms of efficacy of treatment in a given subject. This amount will vary depending upon a variety of factors, including but not limited to the characteristics of the therapeutic compound (including activity, pharmacokinetics, pharmacodynamics, and bioavailability), the physiological condition of the subject (including age, sex, disease type and stage, general physical condition, responsiveness to a given dosage, and type of medication), the nature of the pharmaceutically acceptable carrier or carriers in the formulation, and the route of administration. One skilled in the clinical and pharmacological arts will be able to determine a therapeutically effective amount through routine experimentation, for instance, by monitoring a subject's response to administration of a compound and adjusting the dosage accordingly. For additional guidance, see Remington: The Science and Practice of Pharmacy (Gennaro ed. 20th edition, Williams & Wilkins PA, USA) (2000).

### Biomarkers

Various embodiments of the present invention provide for using miRNAs, DLK1-DIO3 region, and MEG9 as biomarkers for cancer and/or cancer associated tissues.

As such, various embodiments of the present invention provide for a method of predicting responsiveness to a cancer drug, comprising: obtaining a biological sample from a subject; testing the biological sample for a relative increase, decrease, or steady expression of a miRNA; and associating a relative increase of the miRNA expression level with a lower likelihood of drug responsiveness or associating a relative decrease or a steady expression level of the miRNA with a higher likelihood of drug responsiveness. In various embodiments, the method further comprises selecting a cancer drug to administer to the subject when a higher likelihood of drug responsiveness is predicted. In further embodiments, the method comprises administering a selected cancer drug to the subject.

In various embodiments, the cancer drug is a tyrosine kinase inhibitor (TKI). In certain embodiments, the TKI is an EGFR-TKI. In particular embodiments, the EGFR-TKI is Erlotinib (TARCEVA). In particular embodiments, the TKI is Gefitinib. In certain embodiments, the TKI is Apatinib, Cabozantinib, Canertinib, Crenolanib, Damnacanthal, Foretinib, Fostamatinib, Intedanib, Linifanib, Motesanib, Mubritinib, Vatalanib, or Vemurafenib.

In various embodiments, the miRNA is miR-379, miR-379*, miR-193b, miR-193b*, miR-409-5p, miR-409-3p, miR-154, and/or miR-154*. In various embodiments, the miRNA is mature miR-379, miR-379*, miR-193b, miR-193b*, miR-409-5p, miR-409-3p, miR-154, and/or miR-154*. In particular embodiments, the miRNA is mature miR-379, miR-193b, miR-409-5p, miR-409-3p and/or miR-154*. In various embodiments, the miRNA has a sequence as disclosed by SEQ ID NO:2, SEQ ID NO:3, SEQ ID NO:5, SEQ ID NO:6, SEQ ID NO:8, SEQ ID NO:9, SEQ ID NO: 11 or SEQ ID NO:12. In particular embodiments, the miRNA has a sequence as disclosed by SEQ ID NO:2, SEQ ID NO:5, SEQ ID NO: 8, SEQ ID NO:9, or SEQ ID NO: 12.

In particular embodiments, the miRNA is miR-379, miR-379*, miR-154, and/or miR-154*. In various embodiments, the miRNA is mature miR-379, miR-379*, miR-154, and/or miR-154*. In particular embodiments, the miRNA is mature miR-379 and/or miR-154*. In various embodiments, the miRNA has the sequence as disclosed by SEQ ID NO:2, SEQ ID NO:3, SEQ ID NO: 11 or SEQ ID NO:12. In particular embodiments, the miRNA has the sequence as disclosed by SEQ ID NO:2 or SEQ ID NO: 12.

In certain embodiments, the cancer is prostate cancer. In certain embodiments, the cancer is lung cancer. In certain embodiments, the cancer is breast cancer. In certain embodiments, the cancer is metastatic cancer. In certain embodiments, the metastatic cancer is metastasis to the bone. In certain embodiments, the cancer is metastatic prostate cancer. In certain embodiments, the cancer is metastatic lung cancer. In certain embodiments, the cancer is metastatic breast cancer.

Various embodiments of the present invention also provide for a method of detecting a disease state of a cancer in a subject, comprising: obtaining a biological sample from a subject; testing the biological sample for a relative increase, decrease, or steady expression of a miRNA; and associating a relative increase of the miRNA expression level with a higher likelihood of having a cancer drug resistant disease state or associating a relative decrease or a steady expression level of the miRNA with cancer drug susceptible disease state. In various embodiments, the method further comprises selecting a cancer drug to administer to the subject when a cancer drug susceptible disease state is detected. In further embodiments, the method comprises administering a selected cancer drug to the subject.

In various embodiments, the cancer drug is a tyrosine kinase inhibitor (TKI). In certain embodiments, the TKI is an EGFR-TKI. In particular embodiments, the EGFR-TKI is Erlotinib (TARCEVA). In particular embodiments, the TKI is Gefitinib. In certain embodiments, the TKI is Apatinib, Cabozantinib, Canertinib, Crenolanib, Damnacanthal, Foretinib, Fostamatinib, Intedanib, Linifanib, Motesanib, Mubritinib, Vatalanib, or Vemurafenib.

In various embodiments, the miRNA is miR-379, miR-379*, miR-193b, miR-193b*, miR-409-5p, miR-409-3p, miR-154, and/or miR-154*. In various embodiments, the miRNA is mature miR-379, miR-379*, miR-193b, miR-193b*, miR-409-5p, miR-409-3p, miR-154, and/or miR-154*. In particular embodiments, the miRNA is mature miR-379, miR-193b, miR-409-5p, miR-409-3p and/or miR-154*. In various embodiments, the miRNA has a sequence as disclosed by SEQ ID NO:2, SEQ ID NO:3, SEQ ID NO:5, SEQ ID NO:6, SEQ ID NO:8, SEQ ID NO:9, SEQ ID NO: 11 or SEQ ID NO:12. In particular embodiments, the miRNA has a sequence as disclosed by SEQ ID NO:2, SEQ ID NO:5, SEQ ID NO: 8, SEQ ID NO:9, or SEQ ID NO: 12.

In particular embodiments, the miRNA is miR-379, miR-379*, miR-154, and/or miR-154*. In various embodiments, the miRNA is mature miR-379, miR-379*, miR-154, and/or miR-154*. In particular embodiments, the miRNA is mature miR-379 and/or miR-154*. In various embodiments, the miRNA has the sequence as disclosed by SEQ ID NO:2, SEQ ID NO:3, SEQ ID NO: 11 or SEQ ID NO:12. In particular embodiments, the miRNA has the sequence as disclosed by SEQ ID NO:2 or SEQ ID NO: 12.

In certain embodiments, the cancer is prostate cancer. In certain embodiments, the cancer is lung cancer. In certain embodiments, the cancer is breast cancer. In certain embodiments, the cancer is metastatic cancer. In certain embodiments, the metastatic cancer is metastasis to the bone. In certain embodiments, the cancer is metastatic prostate cancer. In certain embodiments, the cancer is metastatic lung cancer. In certain embodiments, the cancer is metastatic breast cancer.

Various embodiments of the present invention also provide for a method of detecting a disease state of a cancer in a subject, comprising: obtaining a biological sample from a subject; testing the biological sample for a relative increase, decrease, or steady expression of an DLK1-miRNA; and associating a relative increased expression the miRNA with metastatic disease state or associating a relative decreased or a steady expression level of DLK1- miRNA with a non-metastatic disease state.

In various embodiments, the miRNA is miR-379, miR-379*, miR-193b, miR-193b*, miR-409-5p, miR-409-3p, miR-154, and/or miR-154*. In various embodiments, the miRNA is mature miR-379, miR-379*, miR-193b, miR-193b*, miR-409-5p, miR-409-3p, miR-154, and/or miR-154*. In particular embodiments, the miRNA is mature miR-379, miR-193b, miR-409-5p, miR-409-3p and/or miR-154*. In various embodiments, the miRNA has a sequence as disclosed by SEQ ID NO:2, SEQ ID NO:3, SEQ ID NO:5, SEQ ID NO:6, SEQ ID NO:8, SEQ ID NO:9, SEQ ID NO: 11 or SEQ ID NO:12. In particular embodiments, the miRNA has a sequence as disclosed by SEQ ID NO:2, SEQ ID NO:5, SEQ ID NO: 8, SEQ ID NO:9, or SEQ ID NO: 12.

In certain embodiments, the metastatic disease state is bone metastasis. In certain embodiments, the cancer is metastatic prostate cancer. In certain embodiments, the cancer is metastatic lung cancer. In certain embodiments, the cancer is metastatic breast cancer.

Various embodiments of the present invention provide for a method of predicting responsiveness to a cancer drug, comprising: obtaining a biological sample from a subject; testing the biological sample for a relative increase, decrease, or steady expression of DLK1-DIO3 cluster/region; and associating a relative increased expression level of the DLK1-DIO3 cluster/region with a lower likelihood of drug responsiveness or associating a relative decreased or a steady expression level of the DLK1-DIO3 cluster/region with a higher likelihood of drug responsiveness. In various embodiments, the method further comprises selecting a cancer drug to administer to the subject when a higher likelihood of drug responsiveness is detected. In further embodiments, the method comprises administering a selected cancer drug to the subject.

In various embodiments, the cancer drug is a tyrosine kinase inhibitor (TKI). In certain embodiments, the TKI is an EGFR-TKI. In particular embodiments, the EGFR-TKI is Erlotinib (TARCEVA). In particular embodiments, the TKI is Gefitinib. In certain embodiments, the TKI is Apatinib, Cabozantinib, Canertinib, Crenolanib, Damnacanthal, Foretinib, Fostamatinib, Intedanib, Linifanib, Motesanib, Mubritinib, Vatalanib, or Vemurafenib.

In certain embodiments, the cancer is prostate cancer. In certain embodiments, the cancer is lung cancer. In certain embodiments, the cancer is breast cancer. In certain embodiments, the cancer is metastatic cancer. In certain embodiments, the metastatic cancer is metastasis to the bone. In certain embodiments, the cancer is metastatic prostate cancer. In certain embodiments, the cancer is metastatic lung cancer. In certain embodiments, the cancer is metastatic breast cancer.

Various embodiments of the present invention also provide for a method of detecting a disease state of a cancer in a subject, comprising: obtaining a biological sample from a subject; testing the biological sample for a relative increase, decrease, or steady expression of the DLK1-DIO3 cluster/region; and associating a relative increased expression level of the DLK1-DIO3 region with a cancer drug resistant disease state or associating a relative decreased or a steady expression level of the DLK1-DIO3 cluster/region with cancer drug susceptible disease state. In various embodiments, the method further comprises selecting a cancer drug to administer to the subject when a cancer drug susceptible disease state is detected. In further embodiments, the method comprises administering a selected cancer drug to the subject.

In various embodiments, the cancer drug is a tyrosine kinase inhibitor (TKI). In certain embodiments, the TKI is an EGFR-TKI. In particular embodiments, the EGFR-TKI is Erlotinib (TARCEVA). In particular embodiments, the TKI is Gefitinib. In certain embodiments, the TKI is Apatinib, Cabozantinib, Canertinib, Crenolanib, Damnacanthal, Foretinib, Fostamatinib, Intedanib, Linifanib, Motesanib, Mubritinib, Vatalanib, or Vemurafenib.

In certain embodiments, the cancer is prostate cancer. In certain embodiments, the cancer is lung cancer. In certain embodiments, the cancer is breast cancer. In certain embodiments, the cancer is metastatic cancer. In certain embodiments, the metastatic cancer is metastasis to the bone. In certain embodiments, the cancer is metastatic prostate cancer. In certain embodiments, the cancer is metastatic lung cancer. In certain embodiments, the cancer is metastatic breast cancer.

Various embodiments of the present invention also provide for a method of detecting a disease state of a cancer in a subject, comprising: obtaining a biological sample from a subject; testing the biological sample for a relative increase, decrease, or steady expression of DLK1-DIO3 cluster/region; and associating a relative increased expression the DLK1-DIO3 cluster/region with metastatic disease state or associating a relative decreased or a steady expression level of DLK1-DIO3 cluster/region with a non-metastatic disease state.

In certain embodiments, the metastatic disease state is bone metastasis. In certain embodiments, the cancer is metastatic prostate cancer. In certain embodiments, the cancer is metastatic lung cancer. In certain embodiments, the cancer is metastatic breast cancer.

Various embodiments of the present invention provide for a method of predicting responsiveness to a cancer drug, comprising: obtaining a biological sample from a subject; testing the biological sample for a relative increase, decrease, or steady expression of MEG9; and associating a relative increased expression level of MEG9 with a lower likelihood of drug responsiveness or associating a relative decreased or a steady expression level of MEG9 with a higher likelihood of drug responsiveness. In various embodiments, the method further comprises selecting a cancer drug to administer to the subject when a higher likelihood of drug responsiveness is detected. In further embodiments, the method comprises administering a selected cancer drug to the subject.

In various embodiments, the cancer drug is a tyrosine kinase inhibitor (TKI). In certain embodiments, the TKI is an EGFR-TKI. In particular embodiments, the EGFR-TKI is Erlotinib (TARCEVA). In particular embodiments, the TKI is Gefitinib. In certain embodiments, the TKI is Apatinib, Cabozantinib, Canertinib, Crenolanib, Damnacanthal, Foretinib, Fostamatinib, Intedanib, Linifanib, Motesanib, Mubritinib, Vatalanib, or Vemurafenib.

In certain embodiments, the cancer is prostate cancer. In certain embodiments, the cancer is lung cancer. In certain embodiments, the cancer is breast cancer. In certain embodiments, the cancer is metastatic cancer. In certain embodiments, the metastatic cancer is metastasis to the bone. In certain embodiments, the cancer is metastatic prostate cancer. In certain embodiments, the cancer is metastatic lung cancer. In certain embodiments, the cancer is metastatic breast cancer.

Various embodiments of the present invention also provide for a method of detecting a disease state of a cancer in a subject, comprising: obtaining a biological sample from a subject; testing the biological sample for a relative increase, decrease, or steady expression of MEG9; and associating a relative increased expression the MEG9 with metastatic disease state or associating a relative decreased or a steady expression level of MEG9 with a non-metastatic disease state.

In certain embodiments, the metastatic disease state is bone metastasis. In certain embodiments, the cancer is metastatic prostate cancer. In certain embodiments, the cancer is metastatic lung cancer. In certain embodiments, the cancer is metastatic breast cancer.

### Systems

Various embodiments of the present invention provide for a system of predicting responsiveness to a cancer drug, comprising: biological sample obtained from a subject; detection probes to test the biological sample for a relative increase, decrease, or steady expression of a miRNA. In various embodiments, the system further comprises a machine (e.g., computer) to associate a relative increase of the miRNA expression level with a lower likelihood of drug responsiveness or associate a relative decrease or a steady expression level of the miRNA with a higher likelihood of drug responsiveness.

In various embodiments, the cancer drug is a tyrosine kinase inhibitor (TKI). In certain embodiments, the TKI is an EGFR-TKI. In particular embodiments, the EGFR-TKI is Erlotinib (TARCEVA). In particular embodiments, the TKI is Gefitinib. In certain embodiments, the TKI is Apatinib, Cabozantinib, Canertinib, Crenolanib, Damnacanthal, Foretinib, Fostamatinib, Intedanib, Linifanib, Motesanib, Mubritinib, Vatalanib, or Vemurafenib.

In various embodiments, the miRNA is miR-379, miR-379*, miR-193b, miR-193b*, miR-409-5p, miR-409-3p, miR-154, and/or miR-154*. In various embodiments, the miRNA is mature miR-379, miR-379*, miR-193b, miR-193b*, miR-409-5p, miR-409-3p, miR-154, and/or miR-154*. In particular embodiments, the miRNA is mature miR-379, miR-193b, miR-409-5p, miR-409-3p and/or miR-154*. In various embodiments, the miRNA has a sequence as disclosed by SEQ ID NO:2, SEQ ID NO:3, SEQ ID NO:5, SEQ ID NO:6, SEQ ID NO:8, SEQ ID NO:9, SEQ ID NO: 11 or SEQ ID NO:12. In particular embodiments, the miRNA has a sequence as disclosed by SEQ ID NO:2, SEQ ID NO:5, SEQ ID NO: 8, SEQ ID NO:9, or SEQ ID NO: 12.

In particular embodiments, the miRNA is miR-379, miR-379*, miR-154, and/or miR-154*. In various embodiments, the miRNA is mature miR-379, miR-379*, miR-154, and/or miR-154*. In particular embodiments, the miRNA is mature miR-379 and/or miR-154*. In various embodiments, the miRNA has the sequence as disclosed by SEQ ID NO:2, SEQ ID NO:3, SEQ ID NO: 11 or SEQ ID NO:12. In particular embodiments, the miRNA has the sequence as disclosed by SEQ ID NO:2 or SEQ ID NO: 12.

In certain embodiments, the cancer is prostate cancer. In certain embodiments, the cancer is lung cancer. In certain embodiments, the cancer is breast cancer. In certain embodiments, the cancer is metastatic cancer. In certain embodiments, the metastatic cancer is metastasis to the bone. In certain embodiments, the cancer is metastatic prostate cancer. In certain embodiments, the cancer is metastatic lung cancer. In certain embodiments, the cancer is metastatic breast cancer.

Various embodiments of the present invention also provide for a system of detecting a disease state of a cancer in a subject, comprising: a biological sample from a subject; detection probes to test the biological sample for a relative increase, decrease, or steady expression of a miRNA. In various embodiments, the system further comprises a machine (e.g., computer) to associate a relative increase of the miRNA expression level with a higher likelihood of having a cancer drug resistant disease state or associate a relative decrease or a steady expression level of the miRNA with cancer drug susceptible disease state.

In various embodiments, the cancer drug is a tyrosine kinase inhibitor (TKI). In certain embodiments, the TKI is an EGFR-TKI. In particular embodiments, the EGFR-TKI is Erlotinib (TARCEVA). In particular embodiments, the TKI is Gefitinib. In certain embodiments, the TKI is Apatinib, Cabozantinib, Canertinib, Crenolanib, Damnacanthal, Foretinib, Fostamatinib, Intedanib, Linifanib, Motesanib, Mubritinib, Vatalanib, or Vemurafenib.

In various embodiments, the miRNA is miR-379, miR-379*, miR-193b, miR-193b*, miR-409-5p, miR-409-3p, miR-154, and/or miR-154*. In various embodiments, the miRNA is mature miR-379, miR-379*, miR-193b, miR-193b*, miR-409-5p, miR-409-3p, miR-154, and/or miR-154*. In particular embodiments, the miRNA is mature miR-379, miR-193b, miR-409-5p, miR-409-3p and/or miR-154*. In various embodiments, the miRNA has a sequence as disclosed by SEQ ID NO:2, SEQ ID NO:3, SEQ ID NO:5, SEQ ID NO:6, SEQ ID NO:8, SEQ ID NO:9, SEQ ID NO: 11 or SEQ ID NO:12. In particular embodiments, the miRNA has a sequence as disclosed by SEQ ID NO:2, SEQ ID NO:5, SEQ ID NO: 8, SEQ ID NO:9, or SEQ ID NO: 12.

In particular embodiments, the miRNA is miR-379, miR-379*, miR-154, and/or miR-154*. In various embodiments, the miRNA is mature miR-379, miR-379*, miR-154, and/or miR-154*. In particular embodiments, the miRNA is mature miR-379 and/or miR-154*. In various embodiments, the miRNA has the sequence as disclosed by SEQ ID NO:2, SEQ ID NO:3, SEQ ID NO: 11 or SEQ ID NO:12. In particular embodiments, the miRNA has the sequence as disclosed by SEQ ID NO:2 or SEQ ID NO:12.

In certain embodiments, the cancer is prostate cancer. In certain embodiments, the cancer is lung cancer. In certain embodiments, the cancer is breast cancer. In certain embodiments, the cancer is metastatic cancer. In certain embodiments, the metastatic cancer is metastasis to the bone. In certain embodiments, the cancer is metastatic prostate cancer. In certain embodiments, the cancer is metastatic lung cancer. In certain embodiments, the cancer is metastatic breast cancer.

Various embodiments of the present invention also provide for a system of detecting a disease state of a cancer in a subject, comprising: a biological sample from a subject; detection probes to test the biological sample for a relative increase, decrease, or steady expression of a DLK1-miRNA. In various embodiments, the system further comprises a machine (e.g., computer) to associate a relative increased expression the miRNA with metastatic disease state or associate a relative decreased or a steady expression level of DLK1- miRNA with a non-metastatic disease state.

In various embodiments, the miRNA is miR-379, miR-379*, miR-193b, miR-193b*, miR-409-5p, miR-409-3p, miR-154, and/or miR-154*. In various embodiments, the miRNA is mature miR-379, miR-379*, miR-193b, miR-193b*, miR-409-5p, miR-409-3p, miR-154, and/or miR-154*. In particular embodiments, the miRNA is mature miR-379, miR-193b, miR-409-5p, miR-409-3p and/or miR-154*. In various embodiments, the miRNA has a sequence as disclosed by SEQ ID NO:2, SEQ ID NO:3, SEQ ID NO:5, SEQ ID NO:6, SEQ ID NO:8, SEQ ID NO:9, SEQ ID NO: 11 or SEQ ID NO:12. In particular embodiments, the miRNA has a sequence as disclosed by SEQ ID NO:2, SEQ ID NO:5, SEQ ID NO: 8, SEQ ID NO:9, or SEQ ID NO: 12.

In certain embodiments, the metastatic disease state is bone metastasis. In certain embodiments, the cancer is metastatic prostate cancer. In certain embodiments, the cancer is metastatic lung cancer. In certain embodiments, the cancer is metastatic breast cancer.

Various embodiments of the present invention provide for a system of predicting responsiveness to a cancer drug, comprising: a biological sample from a subject; detection probes to the biological sample for a relative increase, decrease, or steady expression of DLK1-DIO3 cluster/region. In various embodiments, the system further comprises a machine (e.g., computer) to associate a relative increased expression level of the DLK1-DIO3 cluster/region with a lower likelihood of drug responsiveness or associate a relative decreased or a steady expression level of the DLK1-DIO3 cluster/region with a higher likelihood of drug responsiveness.

In various embodiments, the cancer drug is a tyrosine kinase inhibitor (TKI). In certain embodiments, the TKI is an EGFR-TKI. In particular embodiments, the EGFR-TKI is Erlotinib (TARCEVA). In particular embodiments, the TKI is Gefitinib. In certain embodiments, the TKI is Apatinib, Cabozantinib, Canertinib, Crenolanib, Damnacanthal, Foretinib, Fostamatinib, Intedanib, Linifanib, Motesanib, Mubritinib, Vatalanib, or Vemurafenib.

In certain embodiments, the cancer is prostate cancer. In certain embodiments, the cancer is lung cancer. In certain embodiments, the cancer is breast cancer. In certain embodiments, the cancer is metastatic cancer. In certain embodiments, the metastatic cancer is metastasis to the bone. In certain embodiments, the cancer is metastatic prostate cancer. In certain embodiments, the cancer is metastatic lung cancer. In certain embodiments, the cancer is metastatic breast cancer.

Various embodiments of the present invention also provide for a system of detecting a disease state of a cancer in a subject, comprising: a biological sample from a subject; detection probes to test the biological sample for a relative increase, decrease, or steady expression of the DLK1-DIO3 cluster/region. In various embodiments, the system further comprises a machine (e.g., computer) to associate a relative increased expression level of the DLK1-DIO3 region with a cancer drug resistant disease state or associate a relative decreased or a steady expression level of the DLK1-DIO3 cluster/region with cancer drug susceptible disease state.

In various embodiments, the cancer drug is a tyrosine kinase inhibitor (TKI). In certain embodiments, the TKI is an EGFR-TKI. In particular embodiments, the EGFR-TKI is Erlotinib (TARCEVA). In particular embodiments, the TKI is Gefitinib. In certain embodiments, the TKI is Apatinib, Cabozantinib, Canertinib, Crenolanib, Damnacanthal, Foretinib, Fostamatinib, Intedanib, Linifanib, Motesanib, Mubritinib, Vatalanib, or Vemurafenib.

In certain embodiments, the cancer is prostate cancer. In certain embodiments, the cancer is lung cancer. In certain embodiments, the cancer is breast cancer. In certain embodiments, the cancer is metastatic cancer. In certain embodiments, the metastatic cancer is metastasis to the bone. In certain embodiments, the cancer is metastatic prostate cancer. In certain embodiments, the cancer is metastatic lung cancer. In certain embodiments, the cancer is metastatic breast cancer.

Various embodiments of the present invention also provide for a system of detecting a disease state of a cancer in a subject, comprising: a biological sample from a subject; detection probes to test the biological sample for a relative increase, decrease, or steady expression of DLK1-DIO3 cluster/region. In various embodiments, the system further comprises a machine (e.g., computer) to associate a relative increased expression the DLK1-DIO3 cluster/region with metastatic disease state or associate a relative decreased or a steady expression level of DLK1-DIO3 cluster/region with a non-metastatic disease state.

In certain embodiments, the metastatic disease state is bone metastasis. In certain embodiments, the cancer is metastatic prostate cancer. In certain embodiments, the cancer is metastatic lung cancer. In certain embodiments, the cancer is metastatic breast cancer.

Various embodiments of the present invention provide for a system of predicting responsiveness to a cancer drug, comprising: a biological sample from a subject; detection probes to test the biological sample for a relative increase, decrease, or steady expression of MEG9. In various embodiments, the system further comprises a machine (e.g., computer) to associate a relative increased expression level of MEG9 with a lower likelihood of drug responsiveness or associate a relative decreased or a steady expression level of MEG9 with a higher likelihood of drug responsiveness.

In various embodiments, the cancer drug is a tyrosine kinase inhibitor (TKI). In certain embodiments, the TKI is an EGFR-TKI. In particular embodiments, the EGFR-TKI is Erlotinib (TARCEVA). In particular embodiments, the TKI is Gefitinib. In certain embodiments, the TKI is Apatinib, Cabozantinib, Canertinib, Crenolanib, Damnacanthal, Foretinib, Fostamatinib, Intedanib, Linifanib, Motesanib, Mubritinib, Vatalanib, or Vemurafenib.

In certain embodiments, the cancer is prostate cancer. In certain embodiments, the cancer is lung cancer. In certain embodiments, the cancer is breast cancer. In certain embodiments, the cancer is metastatic cancer. In certain embodiments, the metastatic cancer is metastasis to the bone. In certain embodiments, the cancer is metastatic prostate cancer. In certain embodiments, the cancer is metastatic lung cancer. In certain embodiments, the cancer is metastatic breast cancer.

Various embodiments of the present invention also provide for a system of detecting a disease state of a cancer in a subject, comprising: a biological sample from a subject; detection probes to test the biological sample for a relative increase, decrease, or steady expression of MEG9. In various embodiments, the system further comprises a machine (e.g., computer) to associate a relative increased expression the MEG9 with metastatic disease state or associate a relative decreased or a steady expression level of MEG9 with a non-metastatic disease state.

In certain embodiments, the metastatic disease state is bone metastasis. In certain embodiments, the cancer is metastatic prostate cancer. In certain embodiments, the cancer is metastatic lung cancer. In certain embodiments, the cancer is metastatic breast cancer.

With respect to methods and systems of the present invention, the relative increase, decrease or steady expression of the miRNA, DLK1-miRNA, DLK1-DIO3 cluster/region, or MEG9 is relative to a control (e.g., established control, non-cancerous biological sample, non-cancerous biological sample of the same type (e.g., non-cancerous lung tissue as the control vs. cancerous lung tissue as the biological tested)).

The subjects with respect to the methods and systems of the present invention are subject who have cancer, are suspected of having cancer, are diagnosed with cancer, or suffering from cancer. Examples of cancers are described herein.

Examples of biological sample with respect to methods and systems of the present invention include, but are not limited to mammalian body fluids, sera such as blood (including whole blood as well as its plasma and serum), CSF (spinal fluid), urine, sweat, saliva, tears, pulmonary secretions, breast aspirate, prostate fluid, seminal fluid, stool, cervical scraping, cysts, amniotic fluid, intraocular fluid, mucous, moisture in breath, animal tissue, cell lysates, tumor tissue, hair, skin, buccal scrapings, nails, bone marrow, cartilage, prions, bone powder, ear wax, etc. or even from external or archived sources such as tumor samples (i.e., fresh, frozen or paraffin-embedded).

Examples of detection probes used in the systems or methods of the present invention include nucleic acids, antibodies, a substrate that reacts with the miRNA, DLK1-miRNA, DLK1-DIO3 cluster/region, or MEG9. In various embodiments, the detection probe comprises a label to produce a signal so as to detect the relative increase, decrease or steady expression of the miRNA, DLK1-miRNA, DLK1-DIO3 cluster/region, or MEG9. In various embodiments, the detection probe is in a chip, microarray or gel.

### Drug screening

Various embodiments of the present invention provide for a method of screening for inhibitors or agonists of a miRNA, comprising; providing a test compound; contacting the test compound with a cell expressing the miRNA; detecting a relative increase, decrease, or steady expression of the miRNA; and identifying the test compound as an inhibitor when a relative decrease of the miRNA expression is detected, identifying the test compound as an agonist when a relative increase of the miRNA expression is detected.

In various embodiments, the miRNA is miR-379, miR-379*, miR-193b, miR-193b*, miR-409-5p, miR-409-3p, miR-154, and/or miR-154*. In various embodiments, the miRNA is mature miR-379, miR-379*, miR-193b, miR-193b*, miR-409-5p, miR-409-3p, miR-154, and/or miR-154*. In particular embodiments, the miRNA is mature miR-379, miR-193b, miR-409-5p, miR-409-3p and/or miR-154*. In various embodiments, the miRNA has a sequence as disclosed by SEQ ID NO:2, SEQ ID NO:3, SEQ ID NO:5, SEQ ID NO:6, SEQ ID NO:8, SEQ ID NO:9, SEQ ID NO: 11 or SEQ ID NO:12. In particular embodiments, the miRNA has a sequence as disclosed by SEQ ID NO:2, SEQ ID NO:5, SEQ ID NO: 8, SEQ ID NO:9, or SEQ ID NO: 12.

Various embodiments of the present invention provide for a method of screening for inhibitors or agonists of the DLK1-DIO3 region, comprising; providing a test compound; contacting the test compound with a cell expressing the DLK1-DIO3 region; detecting a relative increase, decrease, or steady expression of the DLK1-DIO3 region; and identifying the test compound as an inhibitor when a relative decrease of the DLK1-DIO3 region expression is detected, identifying the test compound as an agonist when a relative increase of the DLK1-DIO3 region expression is detected.

Various embodiments of the present invention provide for a method of screening for inhibitors or agonists of MEG9, comprising; providing a test compound; contacting the test compound with a cell expressing MEG9; detecting a relative increase, decrease, or steady expression of MEG9; and identifying the test compound as an inhibitor when a relative decrease of MEG9 expression is detected, identifying the test compound as an agonist when a relative increase of MEG9 expression is detected.

The present invention is also directed to kits for practicing various embodiments of the invention. The kit is an assemblage of materials or components, including at least one of the inventive compositions. Thus, in some embodiments the kit contains a composition including miRNA inhibitors as described above. In other embodiments, the kit contains a composition including probes for use in various diagnostic, prognostic, prediction, and/or detection methods and systems.

The exact nature of the components configured in the inventive kit depends on its intended purpose. For example, some embodiments are configured for the purpose of treating cancer. In one embodiment, the kit is configured particularly for the purpose of treating mammalian subjects. In another embodiment, the kit is configured particularly for the purpose of treating human subjects. In further embodiments, the kit is configured for veterinary applications, treating subjects such as, but not limited to, farm animals, domestic animals, and laboratory animals.

Instructions for use may be included in the kit. "Instructions for use" typically include a tangible expression describing the technique to be employed in using the components of the kit to effect a desired outcome, such as to inhibit cancer metastasis. Optionally, the kit also contains other useful components, such as, diluents, buffers, pharmaceutically acceptable carriers, syringes, catheters, applicators, pipetting or measuring tools, bandaging materials or other useful paraphernalia as will be readily recognized by those of skill in the art.

The materials or components assembled in the kit can be provided to the practitioner stored in any convenient and suitable ways that preserve their operability and utility. For example the components can be in dissolved, dehydrated, or lyophilized form; they can be provided at room, refrigerated or frozen temperatures. The components are typically contained in suitable packaging material(s). As employed herein, the phrase "packaging material" refers to one or more physical structures used to house the contents of the kit, such as inventive compositions and the like. The packaging material is constructed by well-known methods, preferably to provide a sterile, contaminant-free environment. As used herein, the term "package" refers to a suitable solid matrix or material such as glass, plastic, paper, foil, and the like, capable of holding the individual kit components. Thus, for example, a package can be a glass vial used to contain suitable quantities of an inventive composition containing miRNA inhibitors. The packaging material generally has an external label which indicates the contents and/or purpose of the kit and/or its components.

### EXAMPLES

The following examples are provided to better illustrate the claimed invention and are not to be interpreted as limiting the scope of the invention. To the extent that specific materials are mentioned, it is merely for purposes of illustration and is not intended to limit the invention. One skilled in the art may develop equivalent means or reactants without the exercise of inventive capacity and without departing from the scope of the invention.

### Example 1

### MicroRNA expression profiling of bone metastatic models of prostate cancer reveal elevated expression of miR-409-5p

MiRNA profiling of two PCa bone metastatic cell based models derived from PCa patients, i.e. the human ARCaP model (ARCaP_{E} and ARCaP_{M}) (E-epithelial, M-mesenchymal), were performed (Fig. 1A) and the LNCaP model (LNCaP and C4-2) (15,16). The ARCaP_{E} and LNCaP are less aggressive and do not metastasize to the bone, whereas the C4-2 and ARCaP_{M} are highly aggressive and metastasize to the bone. Of these cell lines, miR-409-5p, miR-379 and miR-154* are upregulated in the highly bone metastatic ARCaP_{M} cells (Fig. 1D). miR-409-5p is generated from immature transcript and transcribed form 5' end of the pre-miRNA. The ARCaP_{M} cells have 100% bone metastatic ability when injected intra-cardially into mice (17). The ARCaP model is an epithelial to mesenchymal transition model (17). Consistent with previously described EMT associated miRNAs, the miR-200 family members are higher in the epithelial ARCaP_{E} cells compared to the mesenchymal ARCaP_{M} cells (Fig. 1B). miR-409-5p, miR-379 and miR-154* are located in the imprinted DLK1-DIO3 cluster and within a long non-coding RNA, MEG9. Interestingly, MEG9 is also elevated in the bone metastatic ARCaP_{M} prostate cancer cells (Fig. 1C). Several miRNAs from the DLK1-DIO3 cluster were elevated in ARCaP_{M} cells such as, miR-154*, miR-379, miR-487 and miR-409-3p (Fig. 1D). Several miRNAs were down regulated in ARCaP_{M} cells compared to ARCaP_{E} cells, such as miR-495, miR-329, miR-485-3p and miR-299-3p (Fig. 1E). miR-154* and 379 were upregulated in bone metastatic C4-2 cells compared to LNCaP prostate cancer cells. The inventors observed an increase in miR-409-5p in prostate EMT models (Fig. 1D) and lung cancer EMT models (data not shown). The inventors performed a study to determine the expression of miR-409-5p in tumor tissue array (BPH/PIN, n = 23, Gleason score 6-7 , n = 36, Gleason Score 8, n = 24, n= Gleason score 10 n=6) using in situ hybridization and quantum dot detection method (18). miR-409-5p expression was detected in both tumor and tumor cells. miR-409-5p was upregulated in high Gleason score human prostate cancer tissue array, with 100% miR-409-5p positive staining in all Gleason 10 score tissues (Fig. 2A). The negative control (Scramble) had minimal staining and miR-409-5p expression (red) levels were higher in the tumor tissues (Fig. 2C). Nucleus was stained with DAPI.

### Example 2

### Overexpression of miR-409 in normal prostate gland results in tumor growth

To test if miR-409 is oncogenic, the inventors injected human embryonic kidney, 293T cells transfected with miR-409 expressing lentiviral vector carrying green fluorescent protein (GFP) or control vector carrying GFP plasmid (System biosciences). The 293T cells were injected orthotopically into nude mice and tumor development was monitored using tumor specific infrared dye (IR783) (19). The rationale behind this procedure is that lentivirus will be secreted by the producer cells (293T) and infect prostate epithelia and stroma. Strikingly, tumors developed in three to five months in three out of four mice in miR-409 expressing prostates (Fig. 3A). The control mice did not develop tumors. The tumors had green fluorescence and showed tumor specific dye intake (Fig. 3A). Tumor sections were formalin fixed and paraffin embedded and H&E staining was performed. Tumor sections were graded by a pathologist. The tumors varied form benign hyperplasia to adenocarcinoma in miR-409 prostates. The sections were analyzed for miR-409-5p levels. miR-409 expression was observed only in miR-409 expressing prostates and not in control prostates using in situ hybridization and quantum dots (Fig. 3B). Scramble miRNA was used as negative control. Taken together, these studies suggest that, miR-409 expression is sufficient to drive tumor growth in normal prostates, and miR-409 maybe playing an oncogenic role in prostate cancer progression. To the inventors' knowledge this is the first demonstration of an oncogenic miRNA in prostate cancer.

### Example 3

### Inhibition of miR-409-5p in mesenchymal type metastatic prostate cancer cells (ARCaP_{M}) results in cell death, upregulation of miR-409-5p target genes (tumour suppressor) and reversion of Epithelial to mesenchymal transition (EMT to MET)

Inhibition of miR-409-5p using a lentiviral based anti-miR-409-5p resulted in significant cell death of metastatic prostate cancer cells (Fig. 4A). miR-409-5p expression was decreased in ARCaP_{M} cells stably transfected with miR-409-5p inhibitor construct compared to control. miR-409-5p target mRNAs including TUSC4, PHC3 and STAG2 were evaluated and these were increased in miR-409-5p knockdown cells (ARCaP_{M}-409-5pi) metastatic prostate cancer cells compared to the ARCaP_{M} control (ARCaP_{M}-C) cells. TUSC4 is a tumor suppressor protein deleted in solid tumors. PHC3 is part of the polycomb group proteins involved in transcriptional repression and regulation of cell fate (20). The siRNA-mediated down-regulation of TUSC4 induced cell proliferation, while ectopic TUSC4 expression inactivated the PDK1 downstream signaling pathway, including Akt and p70 ribosomal protein S6 kinase, and increased cancer cell sensitivity to several anticancer drugs. Polyhomeotic homolog 3 (PHC 3) is a member of the human polycomb complex and has been regarded as a candidate tumor suppressor of osteosarcoma. PHC3 expression significantly suppressed the colony formation of tumor cells may be caused by modification of the composition of polycomb repressive complex 1 in cancer cells. STAG2 is part of the cohesion complex, where deregulation of the members of the cohesion complex is thought to cause cancer initiation and progression (21). Mutations in STAG2 results in aneuploidy in human cancer. Thus, miR-409 targets are suppressors of oncogenes and thus inhibition of miR-409-5p leads to cell death due to upregulation of such tumor suppressors. When the inventors evaluated the role of other members of the cluster either alone or in combination, it was found that inhibition of miR-379 and miR-154* using a lentiviral based anti-miR-379 and anti-miR-154* or siRNAs that block all three microRNAs (miR-409-5p, miR-379, miR-154*) resulted in significant cell death of metastatic prostate cancer cells (Figure 10).

Both clinical and experimental data support the notion that cancer cells gain their metastatic potential by undergoing EMT. Using a robust ARCaP EMT model, the inventors have previously demonstrated a close association between EMT and prostate cancer bone metastasis. To determine if inhibition of miR-409-5p, miR-379 and miR-154* alone or in combination in mesenchymal metastatic cancer cells could reverse EMT [i.e., induce mesenchymal to epithelial transition (MET)], the inventors performed studies knocking down miR-409-5p, miR-379 and miR-154* sequence-specific siRNA alone or in combination in ARCaP_{M} prostate cancer cells. The control cells were treated similarly, using scrambled siRNA sequence (Scram). miR-409-5p, miR-379 and miR-154* knockdown cells had lower miR-409-5p, miR-379 and miR-154* compared to ARCaP_{M} Scram control. The knockout cells (miRs knocked individually or in combination) underwent stable morphologic mesenchymal to epithelial transition (MET), which was accompanied by increased E-cadherin and decreased vimentin expression. Decreased miR-409-5p, miR-379 and miR-154* also resulted in decreased migration of cancer cells.

### Example 4

### MicroRNA Expression profiling of normal and cancer associated stroma from patients and 3-dimensional cell culture models reveal elevated expression of miR-409-5p levels in the cancer associated stroma from prostate and the bone

Role of miR-409-5p in reactive stroma: In addition to cancer cells, the inventors also performed miRNA profiling of normal and cancer associated stroma in prostate clinical samples and bone stroma models (Fig. 3A).

Prostate stroma: The prostate stromal cells were isolated by laser capture microscopy from patient samples (3). These patient samples had increases in miR-409-5p (Fig. 3B). miR-409-5p was also increased in two out of three patient samples (data not shown).

Bone stroma: Cell based models were used to derive, normal and cancer associated bone osteoblasts cells (normal osteoblast: HS-27a_{RWV}, cancer associated stroma: HS-27_{C4-2}) and osteosarcoma cells (normal: MG_{RWV} and cancer associated: MG_{LN} and MG_{C42}). All the CAFs generated had increased levels of miR-409-5p (Figure 3B). These results demonstrate that stromal cells in addition to cancer cells upregulate miR-409-5p.

### Example 5

### Overexpression of miR-409 in normal prostate stromal cells confers cancer associated stroma-like phenotype through upregulation of bone metastatic markers and downregulation of tumor suppressors

To determine if miR-409 expression in the stroma plays an inductive role in PCa progression, the inventors overexpressed miR-409 in several normal prostate fibroblasts (which do not normally express miR-409) derived from patients (SON and WHN are normal prostate fibroblasts) (Fig. 4A). High levels of miR-409-5p were observed after viral transduction. miR-409 overexpression resulted in increased expression of vimentin and β2-M protein levels (Fig. 4B). Vimentin is a marker for myofibroblasts, which are activated fibroblast (22). miR-409-5p target mRNAs were evaluated and these were decreased in miR-409-expressing normal stromal cells. miR-409-3p mRNA targets were Polyhomeotic protein 3 (PHC3), Tumor suppressor 4 (TUSC4) and RSU1 (Ras suppressor protein 1) as determined by the prediction from TargetScan. These targets are significantly downregulated in miR-409 overexpressing normal stromal cell lines (Fig. 4C). miR-409-5p target also stromal antigen 1 (STAG2), which was significantly downregulated in miR-409 expressing normal stromal cells (Fig. 4D). Inhibition of ras suppressor protein will allow for active signaling through oncogenic ras pathway. Thus, miR-409 targets are suppressors of oncogenes and thus expression of miR-409-5p in normal stroma convert normal fibroblasts to cancer associated fibroblasts.

### Example 6

### Overexpression of miR-409 in normal stroma cells induces aggressive tumorigenicity of cancer cells in mouse models

To determine if miR-409 overexpression in normal fibroblasts (stroma) would induce/enhance cancer cell growth, *in vivo* studies using nude mice were performed. Mice were injected subcutaneously with stromal cells alone (SON-C; control normal stroma), normal stroma expressing miR-409 SON-409), cancer cells alone (C4-2), and tumor stroma combinations (C4-2/SON-C or C4-2/SON-409). Four weeks later, the only group which had tumor growth were those co-injected with C4-2/SON-409 (PCa cells and miR-409 overexpressing stromal cells) (Fig. 5A). The tumor take was 75% in C4-2/SON-409 group (n=4) (Fig. 5A), and had significantly increased tumor volume (Fig. 5B, C). In situ hybridization and quantum dots were used to analyze the expression of miRNA 409-5p and - 3p. H&E was also performed in all tumors. The controls (C4-2, SON-C, SON-409, C4-2/SON-C) had no tumor growth, however the tumor/stroma cells were present in some cases below the skin in the injection site. These tumors were used for miRNA analysis. miR-409-5p expression was only observed in the C4-2/SON-409 tissue sections. Interestingly, miR-409-5p expression was not found only in the stromal cells which overexpressed miR-409, but was also found in the surrounding cancer cells (Fig. 5D). To begin with, C4-2 cells do not express miR-409 and C4-2 tumor cells were not transduced with lentivirus overexpressing miR-409. This suggests that there is a possibility that miR-409 from the stromal cells was secreted externally (exosomal secretion) and taken up by surrounding cancer cells and thus there is an accelerated tumor growth in the C4-2 cancer cells. To the best of the inventors' knowledge, this is the first demonstration of stroma specific expression of a single microRNA conferring aggressiveness to the surrounding tumor to promote cancer progression. These results demonstrate a critical role of miR-409 in the function of cancer associated stroma and in the development of aggressive PCa. Tumor growth was further confirmed by analysis of serum markers such as, prostate specific antigen (PSA) and β2-M secreted proteins. PSA is cancer specific marker, secreted by C4-2 cancer cells alone. β2-M secretion is increased in C4-2/SON-409 group. These results are consistent with *in vitro* studies, where SON-409 stromal cells had increased β2-M levels. High serum β2-M levels is a marker of bone metastasis in PCa (23,24). Interestingly, miR-409 expression is also increased in cancer associated bone stromal cells (Fig. 3B).

### Example 7

### Sequences

**Table 1.**

| **miRNA or inhibitor** | **Sequence (5' - 3')** | **SEQ ID NO.** |
|---|---|---|
| miRZIP379 | 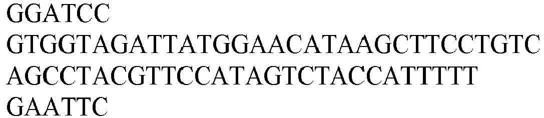 | 1 |
| miR379 mature | ugguagacuauggaacguagg | 2 |
| miR379* mature | uauguaacaugguccacuaacu | 3 |
| miRZIP193b | 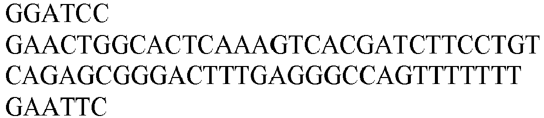 | 4 |
| miR193b-mature | aacuggcccucaaagucccgcu | 5 |
| miR193b*-mature | cgggguuuugagggcgagauga | 6 |
| miRZIP409-5p | 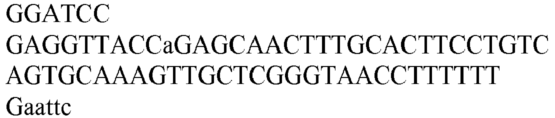 | 7 |
| miR409-5p mature | AGGUUACCCGAGCAACUUUGCAU | 8 |
| miR409-3p-mature | GAAUGUUGCUCGGUGAACCCC U | 9 |
| miRZIP154 | 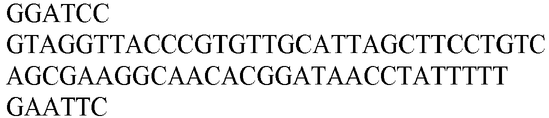 | 10 |
| miR154 mature seq | uagguuauccguguugccuucg | 11 |
| miR154* mature | aaucauacacgguugaccuauu | 12 |

| | | |
|---|---|---|
| miRZIP refers to DNA encoding microRNA inhibitors (short hairpin RNAs directed against mature microRNAs) which cause cell death of prostate cancer cells mature refers to the mature microRNA sequence and cause cancer * refers to the complementary microRNA sequence All miRZIPs are custom made All mature microRNA constructs are purchased from System Biosciences | | |

### Example 8

A prostatic bone growth model was established by intratibial injection of 1 x 10⁶ ARCaPM bone metastatic prostate cancer cells suspended in PBS into both legs of male Nu/Nu nude mice. A week after injection, the mice were treated with vehicle (n = 5) or Vivo Morpholinos (12.5 mg/kg or 25 nmole per injection) (n = 5) alternate days for two weeks. The mode of injection of Morpholinos is intravenous route in the tail vein of mice. Tumor progression was measured using X-ray imaging of bone lesions. Mice were humanely sacrificed on Day 30 and bones were harvested for histopathology.

Targeting microRNAs, for example, miR-409-5p, using Vivo-Morpholinos is expected to reduce bone lesions in mice compared to mice that had been injected with the vehicle (both by X-ray imaging and histopathology studies).

**Table 2.**

| **Morpholino antisense target** | **Morpholino antisense sequence** | **SEQ ID NO.** |
|---|---|---|
| miR379 mature | 5' CGCCTACGTTCCATAGTCTACCATC 3' | 13 |
| miR409-5p mature | 5' GGGTTCACCGAGCAACATTCGTCGT 3' | 14 |
| miR154* mature | 5'AGCGAAGGCAACACGGATAACCTAT 3' | 15 |

### REFERENCES

1. Roodman GD. Mechanisms of bone metastasis. N Engl J Med 2004;350(16):1655-1664.
2. Dakhova O, Ozen M, Creighton CJ, Li R, Ayala G, Rowley D, Ittmann M. Global gene expression analysis of reactive stroma in prostate cancer. Clin Cancer Res 2009;15(12):3979-3989.
3. Sung SY, Hsieh CL, Law A, Zhau HE, Pathak S, Multani AS, Lim S, Coleman IM, Wu LC, Figg WD, Dahut WL, Nelson P, Lee JK, Amin MB, Lyles R, Johnstone PA, Marshall FF, Chung LW. Coevolution of prostate cancer and bone stroma in three-dimensional coculture: implications for cancer growth and metastasis. Cancer Res 2008;68(23):9996-10003.
4. Bhowmick NA, Chytil A, Plieth D, Gorska AE, Dumont N, Shappell S, Washington MK, Neilson EG, Moses HL. TGF-beta signaling in fibroblasts modulates the oncogenic potential of adjacent epithelia. Science 2004;303(5659):848-851.
5. Greenburg G, Hay ED. Epithelia suspended in collagen gels can lose polarity and express characteristics of migrating mesenchymal cells. J Cell Biol 1982;95(1):333-339.
6. Yang J, Weinberg RA. Epithelial-mesenchymal transition: at the crossroads of development and tumor metastasis. Dev Cell 2008;14(6):818-829.
7. Croce CM. Causes and consequences of microRNA dysregulation in cancer. Nat Rev Genet 2009;10(10):704-714.
8. Olive V, Jiang I, He L. mir-17-92, a cluster of miRNAs in the midst of the cancer network. Int J Biochem Cell Biol;42(8): 1348-1354.
9. Valastyan S, Reinhardt F, Benaich N, Calogrias D, Szasz AM, Wang ZC, Brock JE, Richardson AL, Weinberg RA. A pleiotropically acting microRNA, miR-31, inhibits breast cancer metastasis. Cell 2009;137(6):1032-1046.
10. White NM, Fatoohi E, Metias M, Jung K, Stephan C, Yousef GM. Metastamirs: a stepping stone towards improved cancer management. Nat Rev Clin Oncol;8(2):75-84.
11. Sasayama T, Nishihara M, Kondoh T, Hosoda K, Kohmura E. MicroRNA-10b is overexpressed in malignant glioma and associated with tumor invasive factors, uPAR and RhoC. Int J Cancer 2009;125(6):1407-1413.
12. Slaby O, Svoboda M, Fabian P, Smerdova T, Knoflickova D, Bednarikova M, Nenutil R, Vyzula R. Altered expression of miR-21, miR-31, miR-143 and miR-145 is related to clinicopathologic features of colorectal cancer. Oncology 2007;72(5-6):397-402.
13. Lin SL, Chiang A, Chang D, Ying SY. Loss of mir-146a function in hormone-refractory prostate cancer. Rna 2008;14(3):417-424.
14. Peng X, Guo W, Liu T, Wang X, Tu X, Xiong D, Chen S, Lai Y, Du H, Chen G, Liu G, Tang Y, Huang S, Zou X. Identification of miRs-143 and -145 that is associated with bone metastasis of prostate cancer and involved in the regulation of EMT. PLoS One;6(5):e20341.
15. Zhau HY, Chang SM, Chen BQ, Wang Y, Zhang H, Kao C, Sang QA, Pathak SJ, Chung LW. Androgen-repressed phenotype in human prostate cancer. Proc Natl Acad Sci U S A 1996;93(26):15152-15157.
16. Thalmann GN, Anezinis PE, Chang SM, Zhau HE, Kim EE, Hopwood VL, Pathak S, von Eschenbach AC, Chung LW. Androgen-independent cancer progression and bone metastasis in the LNCaP model of human prostate cancer. Cancer Res 1994;54(10):2577-2581.
17. Xu J, Wang R, Xie ZH, Odero-Marah V, Pathak S, Multani A, Chung LW, Zhau HE. Prostate cancer metastasis: role of the host microenvironment in promoting epithelial to mesenchymal transition and increased bone and adrenal gland metastasis. Prostate 2006;66(15):1664-1673.
18. Shi C, Zhou G, Zhu Y, Su Y, Cheng T, Zhau HE, Chung LW. Quantum dots-based multiplexed immunohistochemistry of protein expression in human prostate cancer cells. Eur J Histochem 2008;52(2):127-134.
19. Yang X, Shi C, Tong R, Qian W, Zhau HE, Wang R, Zhu G, Cheng J, Yang VW, Cheng T, Henary M, Strekowski L, Chung LW. Near IR heptamethine cyanine dye-mediated cancer imaging. Clin Cancer Res;16(10):2833-2844.
20. Bracken AP, Helin K. Polycomb group proteins: navigators of lineage pathways led astray in cancer. Nat Rev Cancer 2009;9(11):773-784.
21. Xu H, Tomaszewski JM, McKay MJ. Can corruption of chromosome cohesion create a conduit to cancer? Nat Rev Cancer;11(3):199-210.
22. Wang J, Ying G, Wang J, Jung Y, Lu J, Zhu J, Pienta KJ, Taichman RS. Characterization of phosphoglycerate kinase-1 expression of stromal cells derived from tumor microenvironment in prostate cancer progression. Cancer Res;70(2):471-480.
23. Josson S, Nomura T, Lin JT, Huang WC, Wu D, Zhau HE, Zayzafoon M, Weizmann MN, Gururajan M, Chung LW. beta2-microglobulin induces epithelial to mesenchymal transition and confers cancer lethality and bone metastasis in human cancer cells. Cancer research;71(7):2600-2610.
24. Gross M, Top I, Laux I, Katz J, Curran J, Tindell C, Agus D. Beta-2-microglobulin is an androgen-regulated secreted protein elevated in serum of patients with advanced prostate cancer. Clin Cancer Res 2007;13(7):1979-1986.
25. Chung LW. The role of stromal-epithelial interaction in normal and malignant growth. Cancer surveys 1995;23:33-42.
26. Chung LW. Fibroblasts are critical determinants in prostatic cancer growth and dissemination. Cancer metastasis reviews 1991;10(3):263-274.
27. Rhee HW, Zhau HE, Pathak S, Multani AS, Pennanen S, Visakorpi T, Chung LW. Permanent phenotypic and genotypic changes of prostate cancer cells cultured in a three-dimensional rotating-wall vessel. In vitro cellular & developmental biology 2001;37(3):127-140.
28. Josson S, Matsuoka Y, Chung LW, Zhau HE, Wang R. Tumor-stroma co-evolution in prostate cancer progression and metastasis. Seminars in cell & developmental biology;21(1):26-32.
29. Thalmann GN, Rhee H, Sikes RA, Pathak S, Multani A, Zhau HE, Marshall FF, Chung LW. Human prostate fibroblasts induce growth and confer castration resistance and metastatic potential in LNCaP Cells. European urology;58(1):162-171.
30. Thalmann GN, Sikes RA, Wu TT, Degeorges A, Chang SM, Ozen M, Pathak S, Chung LW. LNCaP progression model of human prostate cancer: androgen-independence and osseous metastasis. The Prostate 2000;44(2):91-103 Jul 101;144(102).
31. Camps JL, Chang SM, Hsu TC, Freeman MR, Hong SJ, Zhau HE, von Eschenbach AC, Chung LW. Fibroblast-mediated acceleration of human epithelial tumor growth in vivo. Proceedings of the National Academy of Sciences of the United States of America 1990;87(1):75-79.
32. Chung LW, Baseman A, Assikis V, Zhau HE. Molecular insights into prostate cancer progression: the missing link of tumor microenvironment. The Journal of urology 2005;173(1):10-20.
33. Zhau HE, Odero-Marah V, Lue HW, Nomura T, Wang R, Chu G, Liu ZR, Zhou BP, Huang WC, Chung LW. Epithelial to mesenchymal transition (EMT) in human prostate cancer: lessons learned from ARCaP model. Clinical & experimental metastasis 2008;25(6):601-610.

Various embodiments of the invention are described above in the Detailed Description. While these descriptions directly describe the above embodiments, it is understood that those skilled in the art may conceive modifications and/or variations to the specific embodiments shown and described herein. Any such modifications or variations that fall within the purview of this description are intended to be included therein as well. Unless specifically noted, it is the intention of the inventors that the words and phrases in the specification and claims be given the ordinary and accustomed meanings to those of ordinary skill in the applicable art(s).

The foregoing description of various embodiments of the invention known to the applicant at this time of filing the application has been presented and is intended for the purposes of illustration and description. The present description is not intended to be exhaustive nor limit the invention to the precise form disclosed and many modifications and variations are possible in the light of the above teachings. The embodiments described serve to explain the principles of the invention and its practical application and to enable others skilled in the art to utilize the invention in various embodiments and with various modifications as are suited to the particular use contemplated. Therefore, it is intended that the invention not be limited to the particular embodiments disclosed for carrying out the invention.

While particular embodiments of the present invention have been shown and described, it will be obvious to those skilled in the art that, based upon the teachings herein, changes and modifications may be made without departing from this invention and its broader aspects and, therefore, the appended claims are to encompass within their scope all such changes and modifications as are within the true spirit and scope of this invention. It will be understood by those within the art that, in general, terms used herein are generally intended as "open" terms *(e.g.,* the term "including" should be interpreted as "including but not limited to," the term "having" should be interpreted as "having at least," the term "includes" should be interpreted as "includes but is not limited to," etc.).

### FURTHER EMBODIMENTS OF THE INVENTION:

1. A method, comprising:
   providing a miRNA inhibitor; and
   administering the miRNA inhibitor to a subject in need of treatment for cancer, in need of treatment for cancer metastasis, or in need of lowering or treatment for cancer drug resistance to treat cancer, to treat cancer metastasis, or to lower or treat cancer drug resistance.
2. The method of embodiment 1, further comprising administering to the subject radiation treatment or chemotherapy treatment.
3. The method of embodiment 1, wherein the cancer is prostate cancer, lung cancer, breast cancer, metastatic cancer, cancer metastasis to the bone, metastatic prostate cancer, metastatic lung cancer, or metastatic breast cancer.
4. The method of embodiment 1, wherein the miRNA inhibitor is capable of inhibiting miR-379, miR-379*, miR-193b, miR-193b*, miR-409-5p, miR-409-3p, miR-154, and/or miR-154*.
5. The method of embodiment 1, wherein the miRNA inhibitor is capable of inhibiting mature miR-379, miR-379*, miR-193b, miR-193b*, miR-409-5p, miR-409-3p, miR-154, and/or miR-154*.
6. The method of embodiment 1, wherein the miRNA inhibitor is a shRNA directed against a mature miRNA.
7. The method of embodiment 1, wherein the miRNA inhibitor is a siRNA directed against a mature miRNA.
8. The method of embodiment 1, wherein the miRNA inhibitor is encoded by a polynucleotide as disclosed by SEQ ID NO:1, SEQ ID NO:4, SEQ ID NO:7, or SEQ ID NO:10, and administering comprises administering the polynucleotide.
9. The method of embodiment 1, wherein the miRNA inhibitor is a shRNA or a siRNA capable of interfering the expression of SEQ ID NO:2, SEQ ID NO:3, SEQ ID NO:5, SEQ ID NO:6, SEQ ID NO:8, SEQ ID NO:9, SEQ ID NO: 11 or SEQ ID NO: 12.
10. A method, comprising:
   obtaining a biological sample from a subject;
   testing the biological sample for a relative increase, decrease, or steady expression of a miRNA; and
   associating a relative increase of the miRNA expression level with a lower likelihood of cancer drug responsiveness or associating a relative decrease or a steady expression level of the miRNA with a higher likelihood of drug responsiveness,
   associating a relative increase of the miRNA expression level with a higher likelihood of having a cancer drug resistant disease state or associating a relative decrease or a steady expression level of the miRNA with cancer drug susceptible disease state, or
   associating a relative increased expression the DLK1-miRNA with metastatic disease state or associating a relative decreased or a steady expression level of DLK1-miRNA with a non-metastatic disease state.
11. The method of embodiment 10, further comprising selecting a cancer drug to administer to the subject when a higher likelihood of drug responsiveness or a cancer drug susceptible disease state is detected.
12. The method of embodiment 10, wherein the drug is a tyrosine kinase inhibitor (TKI).
13. The method of embodiment 11, wherein the TKI is Erlotinib, Gefitinib, Apatinib, Cabozantinib, Canertinib, Crenolanib, Damnacanthal, Foretinib, Fostamatinib, Intedanib, Linifanib, Motesanib, Mubritinib, Vatalanib, or Vemurafenib.
14. The method of embodiment 10, wherein miRNA is miR-379, miR-379*, miR-193b, miR-193b*, miR-409-5p, miR-409-3p, miR-154, miR-154*, mature miR-379, mature miR-379*, mature miR-193b, mature miR-193b*, mature miR-409-5p, mature miR-409-3p, mature miR-154, mature miR-154*, SEQ ID NO:2, SEQ ID NO:3, SEQ ID NO:5, SEQ ID NO:6, SEQ ID NO:8, SEQ ID NO:9, SEQ ID NO: 11 or SEQ ID NO:12.
15. The method of embodiment 10, wherein the cancer is prostate cancer, lung cancer, breast cancer, metastatic cancer, cancer metastasis to the bone, metastatic prostate cancer, metastatic lung cancer or metastatic breast cancer.
16. A method, comprising:
   obtaining a biological sample from a subject;
   testing the biological sample for a relative increase, decrease, or steady expression of DLK1-DIO3 cluster/region; and
   associating a relative increased expression level of the DLK1-DIO3 cluster/region with a lower likelihood of drug responsiveness or associating a relative decreased or a steady expression level of the DLK1-DIO3 cluster/region with a higher likelihood of drug responsiveness,
   associating a relative increased expression level of the DLK1-DIO3 region with a cancer drug resistant disease state or associating a relative decreased or a steady expression level of the DLK1-DIO3 cluster/region with cancer drug susceptible disease state, or
   associating a relative increased expression the DLK1-DIO3 cluster/region with metastatic disease state or associating a relative decreased or a steady expression level of DLK1-DIO3 cluster/region with a non-metastatic disease state.
17. The method of embodiment 16, further comprising selecting a cancer drug to administer to the subject when a higher likelihood of drug responsiveness or a cancer drug susceptible disease state is detected.
18. The method of embodiment 16, wherein the drug is a tyrosine kinase inhibitor (TKI).
19. The method of embodiment 18, wherein the TKI is Erlotinib, Gefitinib, Apatinib, Cabozantinib, Canertinib, Crenolanib, Damnacanthal, Foretinib, Fostamatinib, Intedanib, Linifanib, Motesanib, Mubritinib, Vatalanib, or Vemurafenib.
20. The method of embodiment 16, wherein the disease state is prostate cancer, lung cancer, breast cancer, metastatic cancer, cancer metastasis to the bone, metastatic prostate cancer, metastatic lung cancer or metastatic breast cancer.
21. A method, comprising:
   obtaining a biological sample from a subject;
   testing the biological sample for a relative increase, decrease, or steady expression of MEG9; and
   associating a relative increased expression level of MEG9 with a lower likelihood of drug responsiveness or associating a relative decreased or a steady expression level of MEG9 with a higher likelihood of cancer drug responsiveness.
22. The method of claim 21, further comprising selecting a cancer drug to administer to the subject when a higher likelihood of drug responsiveness.
23. The method of claim 21, wherein the drug is a tyrosine kinase inhibitor (TKI).
24. The method of embodiment 23, wherein the TKI is Erlotinib, Gefitinib, Apatinib, Cabozantinib, Canertinib, Crenolanib, Damnacanthal, Foretinib, Fostamatinib, Intedanib, Linifanib, Motesanib, Mubritinib, Vatalanib, or Vemurafenib.
25. A method, comprising:
   obtaining a biological sample from a subject;
   testing the biological sample for a relative increase, decrease, or steady expression of MEG9; and
   associating a relative increased expression the MEG9 with metastatic disease state or associating a relative decreased or a steady expression level of MEG9 with a non-metastatic disease state.
26. The method of embodiment 25, wherein the disease state is prostate cancer, lung cancer, breast cancer, metastatic cancer, cancer metastasis to the bone, metastatic prostate cancer, metastatic lung cancer or metastatic breast cancer.
27. A system, comprising:
   a biological sample from a subject; and
   a probe to test the biological sample for a relative increase, decrease, or steady expression of a miRNA.
28. The system of embodiment 27, further comprising a machine to
   associate a relative increase of the miRNA expression level with a lower likelihood of drug responsiveness or associating a relative decrease or a steady expression level of the miRNA with a higher likelihood of cancer drug responsiveness,
   associate a relative increase of the miRNA expression level with a higher likelihood of having a cancer drug resistant disease state or associating a relative decrease or a steady expression level of the miRNA with cancer drug susceptible disease state, or
   associate a relative increased expression the DLK1-miRNA with metastatic disease state or associating a relative decreased or a steady expression level of DLK1-miRNA with a non-metastatic disease state.
29. The system of embodiment 27, wherein miRNA is miR-379, miR-379*, miR-193b, miR-193b*, miR-409-5p, miR-409-3p, miR-154, miR-154*, mature miR-379, mature miR-379*, mature miR-193b, mature miR-193b*, mature miR-409-5p, mature miR-409-3p, mature miR-154, mature miR-154*, SEQ ID NO:2, SEQ ID NO:3, SEQ ID NO:5, SEQ ID NO:6, SEQ ID NO:8, SEQ ID NO:9, SEQ ID NO: 11 or SEQ ID NO:12.
30. The system of embodiment 28, wherein the cancer or disease state is prostate cancer, lung cancer, breast cancer, metastatic cancer, cancer metastasis to the bone, metastatic prostate cancer, metastatic lung cancer or metastatic breast cancer.
31. A system, comprising:
   a biological sample from a subject; and
   a probe to test the biological sample for a relative increase, decrease, or steady expression of DLK1-DIO3 cluster/region.
32. The system of embodiment 31, further comprising a machine to
   associate a relative increased expression level of the DLK1-DIO3 cluster/region with a lower likelihood of drug responsiveness or associating a relative decreased or a steady expression level of the DLK1-DIO3 cluster/region with a higher likelihood of drug responsiveness,
   associate a relative increased expression level of the DLK1-DIO3 region with a cancer drug resistant disease state or associating a relative decreased or a steady expression level of the DLK1-DIO3 cluster/region with cancer drug susceptible disease state, or
   associate a relative increased expression the DLK1-DIO3 cluster/region with metastatic disease state or associating a relative decreased or a steady expression level of DLK1-DIO3 cluster/region with a non-metastatic disease state.
33. The system of embodiment 32, wherein the cancer or disease state is prostate cancer, lung cancer, breast cancer, metastatic cancer, cancer metastasis to the bone, metastatic prostate cancer, metastatic lung cancer or metastatic breast cancer.
34. A system, comprising:
   a biological sample from a subject; and
   a probe to test the biological sample for a relative increase, decrease, or steady expression of MEG9.
35. The system of embodiment 34, further comprising a machine to associate a relative increased expression level of MEG9 with a lower likelihood of drug responsiveness or associate a relative decreased or a steady expression level of MEG9 with a higher likelihood of cancer drug responsiveness.
36. A system, comprising:
   a biological sample from a subject; and
   a probe to test the biological sample for a relative increase, decrease, or steady expression of MEG9.
37. The system of embodiment 36, further comprising a machine to associate a relative increased expression the MEG9 with metastatic disease state or associate a relative decreased or a steady expression level of MEG9 with a non-metastatic disease state.
38. The system of embodiment 37, wherein the disease state is prostate cancer, lung cancer, breast cancer, metastatic cancer, cancer metastasis to the bone, metastatic prostate cancer, metastatic lung cancer or metastatic breast cancer.
39. A method, comprising:
   providing a test compound;
   contacting the test compound with a cell expressing a miRNA;
   detecting a relative increase, decrease, or steady expression of the miRNA; and
   identifying the test compound as an inhibitor when a relative decrease of the miRNA expression is detected, or identifying the test compound as an agonist when a relative increase of the miRNA expression is detected.
40. The method of embodiment 39, wherein miRNA is miR-379, miR-379*, miR-193b, miR-193b*, miR-409-5p, miR-409-3p, miR-154, miR-154*, mature miR-379, mature miR-379*, mature miR-193b, mature miR-193b*, mature miR-409-5p, mature miR-409-3p, mature miR-154, mature miR-154*, SEQ ID NO:2, SEQ ID NO:3, SEQ ID NO:5, SEQ ID NO:6, SEQ ID NO:8, SEQ ID NO:9, SEQ ID NO: 11 or SEQ ID NO:12.
41. A method, comprising:
   providing a test compound;
   contacting the test compound with a cell expressing the DLK1-DIO3 region;
   detecting a relative increase, decrease, or steady expression of the DLK1-DIO3 region; and
   identifying the test compound as an inhibitor when a relative decrease of the DLK1-DIO3 region expression is detected, or identifying the test compound as an agonist when a relative increase of the DLK1-DIO3 region expression is detected.
42. A method, comprising:
   providing a test compound;
   contacting the test compound with a cell expressing MEG9;
   detecting a relative increase, decrease, or steady expression of MEG9; and
   identifying the test compound as an inhibitor when a relative decrease of MEG9 expression is detected, or identifying the test compound as an agonist when a relative increase of MEG9 expression is detected.

## Claims

1. An inhibitor of an miRNA of the DLK1-DIO3 cluster/region for use in treating a subject in need of treatment for cancer, in need of treatment for cancer metastasis, or in need of lowering or treatment for cancer drug resistance to treat cancer, to treat cancer metastasis, or to lower or treat cancer drug resistance.

2. The inhibitor of claim 1 for use in treating subjects receiving radiation treatment or chemotherapy treatment.

3. The inhibitor of claim 1 for use in treating subjects with prostate cancer, lung cancer, breast cancer, metastatic cancer, cancer metastasis to the bone, metastatic prostate cancer, metastatic lung cancer, or metastatic breast cancer.

4. The inhibitor of claim 1, wherein the miRNA inhibitor is capable of inhibiting miR-409-5p, miR-409-3p, miR-154*, miR-379, miR-379*, miR-193b, miR-193b*and/or miR-154.

5. The inhibitor of claim 1, wherein the miRNA inhibitor is a shRNA, siRNA or antisense morpholino directed against a mature miRNA.

6. The inhibitor of claim 1, wherein the miRNA inhibitor is encoded by a polynucleotide as disclosed by SEQ ID NO:7, SEQ ID NO:1, SEQ ID NO:4, or SEQ ID NO:10, or is an antisense morpholino as disclosed by SEQ ID NO: 14, SEQ ID NO: 13 or SEQ ID NO: 15.

7. The inhibitor of claim 1, wherein the miRNA inhibitor is a shRNA, a siRNA or an antisense morpholino capable of interfering the expression of SEQ ID NO:8, SEQ ID NO:2, SEQ ID NO:3, SEQ ID NO:5, SEQ ID NO:6, SEQ ID NO:9, SEQ ID NO:11 or SEQ ID NO: 12.

8. A method, comprising:
testing a biological sample obtained from a subject for a relative increase, decrease, or steady expression of a miRNA or of the DLK1-DIO3 cluster/region; and
associating a relative increase of the miRNA or of the DLK1-DIO3 cluster/region expression level with a higher likelihood of having a cancer drug resistant disease state or associating a relative decrease or a steady expression level of the miRNA or of the DLK1-DIO3 cluster/region with cancer drug susceptible disease state,
associating a relative increased expression the miRNA or of the DLK1-DIO3 cluster/region with metastatic disease state or associating a relative decreased or a steady expression level of miRNA or of the DLK1-DIO3 cluster/region with a non-metastatic disease state, or
associating a relative increase of the miRNA or of the DLK1-DIO3 cluster/region expression level with a lower likelihood of cancer drug responsiveness or associating a relative decrease or a steady expression level of the miRNA or of the DLK1-DIO3 cluster/region with a higher likelihood of drug responsiveness.

9. The method of claim 8 wherein MEG 9 expression within the DLK1-DIO3 cluster/region is tested and associating a relative increased expression of MEG9 with metastatic disease state or associating a relative decreased or a steady expression level of MEG9 cluster/region with a non-metastatic disease state.

10. The method of claim 8 or 9, wherein the cancer is prostate cancer, lung cancer, breast cancer, metastatic cancer, cancer metastasis to the bone, metastatic prostate cancer, metastatic lung cancer or metastatic breast cancer.

11. The method of claim 8 wherein MEG 9 expression within the DLK1-DIO3 cluster/region is tested and associating a relative increased expression level of MEG9 with a lower likelihood of drug responsiveness or associating a relative decreased or a steady expression level of MEG9 with a higher likelihood of cancer drug responsiveness.

12. The method of claim 8 or 11, further comprising selecting a cancer drug to administer to the subject when a higher likelihood of drug responsiveness or a cancer drug susceptible disease state is detected.

13. The method of claim 12, wherein the drug is a tyrosine kinase inhibitor (TKI).

14. The method of claim 13, wherein the TKI is Erlotinib, Gefitinib, Apatinib, Cabozantinib, Canertinib, Crenolanib, Damnacanthal, Foretinib, Fostamatinib, Intedanib, Linifanib, Motesanib, Mubritinib, Vatalanib, or Vemurafenib.

15. The method of claim 8, wherein miRNA is tested and the miRNA is miR-409-5p, miR-409-3p, miR-154*, miR-379, miR-379*, miR-193b, miR-193b*, miR-154, mature miR-409-3p, mature miR-409-5p, mature miR-154*, mature miR-379, mature miR-379*, mature miR-193b, mature miR-193b*, mature miR-154, SEQ ID NO:2, SEQ ID NO:3, SEQ ID NO:5, SEQ ID NO:6, SEQ ID NO:8, SEQ ID NO:9, SEQ ID NO: 11 or SEQ ID NO:12.
